# EUROPEAN PATENT SPECIFICATION

(11) **EP 3 880 111 B1**
(45) Date of publication and mention of the grant of the patent: **02.08.2023**
(21) Application number: 19817043.3
(22) Date of filing: 13.11.2019
(51) Int. Cl.: A61C 7/08, A61C 19/045, A61C 17/22, A61F 5/56

(54) **IMPACT SENSING MOUTHGUARD AND METHOD OF PRODUCING THE SAME**
AUFPRALLERFASSENDER MUNDSCHUTZ UND HERSTELLUNGSMETHOD DIESES MUNDSCHUTZES
PROTÈGE-DENTS À DÉTECTION D'IMPACT ET MÉTHODE DE FABRICATION DE CE PROTÈGE-DENTS

(30) Priority: 13.11.2018 US 201862760512 P
(43) Date of publication of application: 22.09.2021
(73) Proprietor: Prevent Biometrics, Inc., Edina, MN 55435 (US); Maloney, Kyle Anthony, Stillwater MN 55082 (US); Larsen, Christopher Scott, Plymouth MN 55447 (US); Washburn, Steven J., Hayward WI 54843 (US)
(72) Inventor: MALONEY, Kyle Anthony, Stillwater, Minnesota 55082 (US); LARSEN, Christopher Scott, Plymouth, Minnesota 55447 (US); WASHBURN, Steven J., Hayward, Wisconsin 54843 (US)
(74) Representative: Murgitroyd & Company
(86) International application number: PCT/US2019/061215
(87) International publication number: WO 2020/102378

(56) References cited:
- WO-A1-2016/168939
- JP-A- 2000 070 292
- US-A1- 2012 172 677
- US-A1- 2015 173 856
- US-B1- 9 968 777

## Description

### CROSS-REFERENCE TO RELATED APPLICATIONS

### TECHNOLOGICAL FIELD

The present disclosure relates to oral appliances such as retainers, dentures, mouthpieces and/or mouthguards. More particularly, the present disclosure relates to an oral appliance having circuitry secured thereon, such as by embedding, incorporating, lodged, attaching, or otherwise securing the circuitry. Still more particularly, the present disclosure relates to an impact-sensing oral appliance having circuitry secured thereon that is configured for sensing and analyzing impacts to the wearer and communicating associated data and/or results.

### BACKGROUND

The background description provided herein is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

Impact data relating to head impacts or other motion data occurring during sports, military activities, exercise, or other activities has become valuable for monitoring and management purposes. This data is valuable for, among other things, monitoring and managing injuries, particularly head injuries. Given a focus on head injuries, benefits of using a mouthguard for sensing the motion data has been realized because the upper jaw is integral with the skull. A mouthguard may be coupled to the upper teeth allowing for very accurate skull motion data to be captured. The motion data may be analyzed to determine and assess impacts to the head or other motion data.

Embedding circuitry in mouthguards in a way that provides reliable performance comes with a whole host of difficulties. First, mouthguards may often be constructed using an injection molding process. The heats, pressures, and limited flow control of injection molding can lead to many problems with so called smart mouthguards. That is, for example, injection molding temperatures may often range from 60-220 °C and pressures may exceed 10,000 psi. Unfortunately, circuitry including batteries, accelerometers, gyroscopes, wiring, chips, soldered circuit boards, and other relatively delicate computer components are not well suited for exposure to such temperatures and pressures. Adhesives and other securing components, electrical connections, and/or the components themselves may be damaged or otherwise fail at these temperatures and pressures. Moreover, when circuitry is placed in an injection molding process, injected material may work its way around, within, and/or into the computer components in a way that may interrupt functionality and/or damage components.

Second, mouthguard use can be a very harsh environment. In some cases, customized mouthguards may be put through a boil/bite process where the mouthguard is heated to a temperature allowing the material of the mouthguard to soften and become formable. The user may place the mouthguard in their mouth and bite the mouthguard to form it to their teeth. The temperatures experienced by the mouthguard during this process can be damaging to circuitry. Moreover, when used as intended, mouthguards fit between the upper and lower teeth of a user and are subject to high biting forces in a moist and/or wet environment including human saliva and other liquids containing a wide variety of fluids and/or chemicals. This alone creates issues of sealing of the mouthguard to prevent moisture from reaching the circuitry. To make matters worse, however, users of mouthguards often chew on mouthguards at angles inconsistent with the intended bite direction. This has a tendency to distort, warp, and/or crush portions of the mouthguard, which can damage the mouthguard and its components. Outside of the mouth, mouthguards may be attached to tether and secured to a helmet for example. As such, the mouthguard may be whipped around and may slam, run into, or otherwise impact the helmet or surrounding objects. Mouthguards may be stored in gym bags and may be subject to bouncing against other items in the bag and/or crushing forces from other items in the bag, stacked bags, or other mishandling of bags and equipment.

US 2015/173856 A1 and WO 2016/168939 A1 disclose oral appliances comprising an oral tray and circuitry components which are respectively embedded in the oral tray and glued to the oral tray.

### SUMMARY

The present invention provides an oral appliance and a method of manufacturing an oral appliance as defined in the claims.

While multiple embodiments are disclosed, still other embodiments of the present disclosure will become apparent to those skilled in the art from the following detailed description, which shows and describes illustrative embodiments of the invention. As will be realized, the various embodiments of the present disclosure are capable of modifications in various obvious aspects, all without departing from the scope of the present disclosure as defined by the appended claims. Accordingly, the drawings and detailed description are to be regarded as illustrative in nature and not restrictive.

### BRIEF DESCRIPTION OF THE DRAWINGS

While the specification concludes with claims particularly pointing out and distinctly claiming the subject matter that is regarded as forming the various embodiments of the present disclosure, it is believed that the invention will be better understood from the following description taken in conjunction with the accompanying Figures, in which:
FIG. 1 is a front and side perspective view of an oral appliance, according to one or more embodiments.
FIG. 2 is rear and side perspective view thereof.
FIG. 3 is an exploded view thereof.
FIG. 4 is a front and side perspective view of a base portion of the oral appliance of FIG. 1.
FIG. 5 is a rear and side perspective view thereof.
FIG. 6 is a rear and side perspective view of circuitry of the oral appliance of FIG. 1.
FIG. 7 is a front and side perspective view thereof.
FIG. 8 is a front and side perspective view of a cover portion of the oral appliance of FIG. 1.
FIG. 9 is a rear perspective view thereof.
FIG. 10 is a front and side perspective view of a dentition portion of the oral appliance of FIG. 1.
FIG. 11 is another front and side perspective view thereof.
FIG. 12 is a partial perspective view of the oral appliance of FIG. 1 showing unintended lateral bite forces experienced by the oral appliance, according to one or more embodiments.
FIG. 13 is a front and side perspective view of an oral appliance, according to one or more embodiments.
FIG. 14 is rear and side perspective view thereof.
FIG. 15 is an exploded view thereof.
FIG. 16 is a front and side perspective view of a base portion of the oral appliance of FIG. 13.
FIG. 17 is a rear and side perspective view thereof.
FIG. 18 is a rear and side perspective view of circuitry of the oral appliance of FIG. 13.
FIG. 19 is a front and side perspective view thereof.
FIG. 20 is a front and side perspective view of a cover portion of the oral appliance of FIG. 13.
FIG. 21 is a rear perspective view thereof.
FIG. 22 is a front and side perspective view of a dentition portion of the oral appliance of FIG. 13.
FIG. 23 is another front and side perspective view thereof.
FIG. 24 is a perspective view of an oral appliance and depicting the difficulty of inadvertently triggering an in-the-mouth or on teeth reading, according to one or more embodiments.
FIG. 25 is a perspective view of an oral appliance in an individual charging case, according to one or more embodiments.
FIG. 26 is a perspective view of a charging case for multiple oral appliances, according to one or more embodiments.
FIG. 27 is a partial cross-sectional view thereof.
FIG. 28 is an internal view of the charging case of FIG. 26 showing power distribution bus boards, according to one or more embodiments.
FIG. 29 is a diagram of a method of manufacturing an oral appliance, according to one or more embodiments.

### DETAILED DESCRIPTION

The present disclosure, in one or more embodiments, relates to an impact sensing oral appliance. The oral appliance may include a mouthguard that is particularly designed to include circuitry and to protect that circuitry from damage during manufacturing and during use. In particular, the mouthguard may include a relatively thick and robust base portion having pockets or recesses for receiving and protecting portions of the circuitry. The circuitry itself may include relatively rigid board portions linked together with flexible portions. The board portions may close off the pockets or recesses and, together with the base portion, function to protect the portions of the circuitry positioned in the pockets. Coatings may be applied to the circuitry to resist damage due to moisture, corrosion, and/or molding fluids. Additionally or alternatively, the board portions may be sealed at the peripheral edge of the pockets to prevent access of moisture or molding fluids into the pockets or recesses and to the circuitry arranged therein. All or a portion of the circuitry may face inward away from the outer environment and toward the teeth to further protect the circuitry from damage due to impacts or inappropriate and/or unintended use, such as lateral biting. The circuitry being backed by rigid board portions that may further protect the circuitry from damage due to lateral biting. In one or more embodiments, the oral appliance may be a custom mouthguard that is individualized to fit a single person.

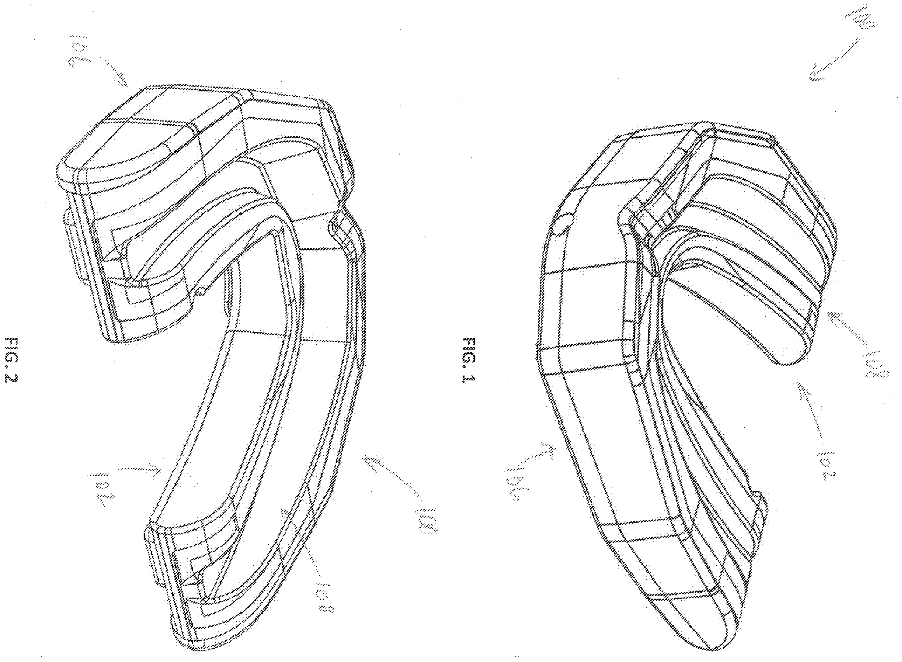

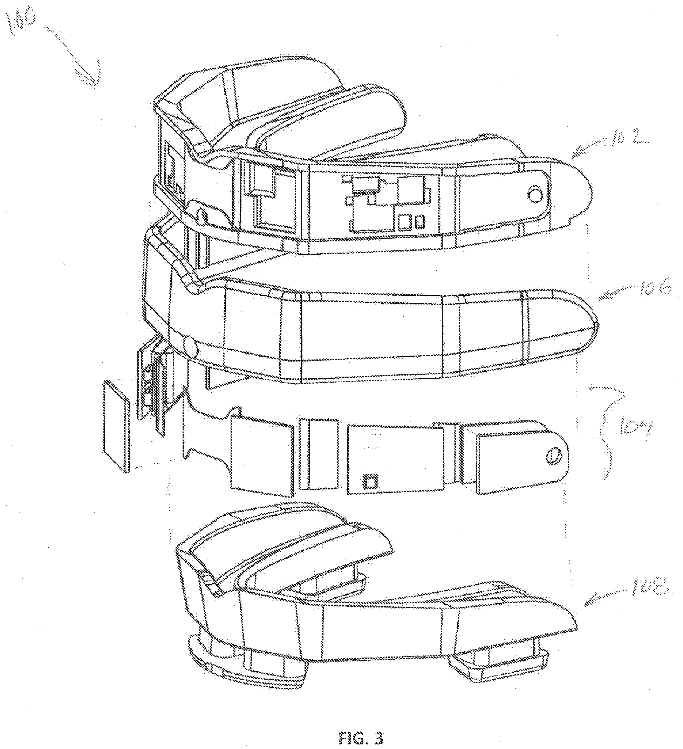

Turning now to FIGS. 1 and 2, an oral appliance 100 is shown. While an oral appliance in the form of a mouthguard is shown, the oral appliance may also include a retainer, a denture or other dentition, a mouthpiece, or other devices for placing in and/or around the mouth. The oral appliance shown may be a mouthguard that is particularly adapted for protecting the teeth of a user during activity and, as such, may include a relatively soft upper portion for engaging the upper teeth and a relatively soft lower portion for engaging the lower teeth. The relatively soft upper portion may be particularly adapted for conforming to the upper teeth to provide a high level of coupling to the upper teeth. In addition, the oral appliance may also be adapted to sense motion of the user and, in particular, the user's upper teeth which are substantially integral with the skull of the user. As such, the oral appliance may include embedded circuitry adapted to sense motion, analyze the motion, and communicate data associated with the motion. In one or more embodiments, and as shown in FIG. 3, the oral appliance 100 may include a base portion 102, circuitry 104, a cover portion 106, and a dentition portion 108.

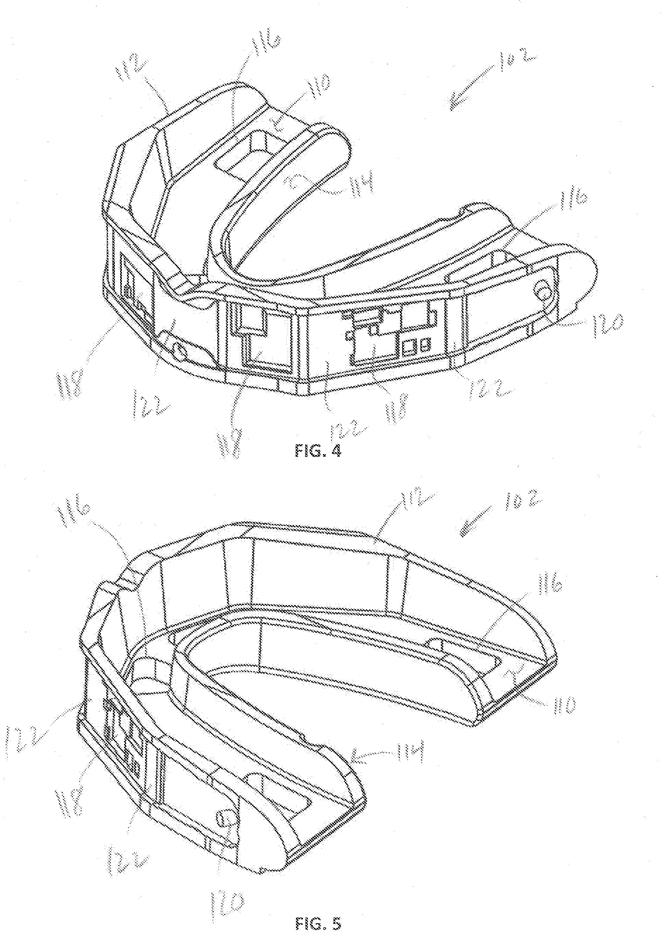

With reference to FIGS. 4 and 5, a base portion 102 is shown. The base portion 102 may form a foundational core of the oral appliance 100 that is configured to define the overall general shape and size of the mouthguard and provide a foundation upon which the remaining portions of the mouthguard may be arranged, secured, and/or placed. As shown, the base portion may include a bite platform, tray, or shelf 110, a labial flange 112, and a lingual flange 114.

The bite platform, tray, or shelf 110 may be configured for generally resting between the upper and lower teeth of a user. As such, the bite platform, tray, or shelf 110 may include a substantially u-shaped element that is substantially flat and has a substantially uniform thickness. The bite platform 110 may be sized for a particular user or a generally sized platform may be provided for a selected range of users and mouth sizes. In one or more embodiments, the bite platform 110 may be adapted to extend along an arc from a front of a user's mouth near the front central incisors rearwardly to the user's 2^{nd}, or 3^{rd} molars, for example. Various sizes of the bite platform 110 may be provided to accommodate a wide variety of mouth sizes including junior and adult sizes. The bite platform 110 may have a varying width to accommodate wider teeth near the back of the user's mouth and narrower teeth near the front of a user's mouth and as such, the bite platform may taper from back to front around the u-shape. To provide a reasonable amount of rigidity, the bite platform may have a thickness ranging from approximately 1 mm to 6 mm or from approximately 2 mm to 4 mm or from approximately 3 mm to 3.5 mm. Still other thicknesses of the bite platform 110 may be provided. In one or more embodiments, the bite platform may include one or more passthrough openings 116. As shown, the bite platform 110 may include rear openings and front openings. The openings 116 may be adapted to allow dentition material to flow through the openings 116 and provide continuity between the upper and lower portions of the dentition element 108 described in more detail below. In one or more embodiments, the passthrough openings 116 may be round, rectangular, square, or other shaped openings.

While not shown, the bite platform 110 may include dentition features particularly adapted to fit the teeth of a particular user. That is, for example, where dentition data is available, a mold of a user's teeth may be used to injection mold and/or 3D print a bite platform 110 that is sized, shaped, and detailed to fit a user's teeth and teeth features. In this embodiment, a softer material may be used for the base portion 102, for example EVA. In addition, the passthrough openings 116 may be omitted and the dentition material 108 may also be omitted. In still another embodiment, the bite platform 110 may be provided as shown in the Figures and the dentition portion of the mouthguard may be pre-formed using a model of the user's teeth and an injection molding or 3D print process to create a dentition portion that is pre-fitted to a user's teeth such that a boil/bite process may be avoided or omitted. Still other approaches to creating a suitable dentition portion may be provided and are discussed in more detail below with respect to the dentition portion 108.

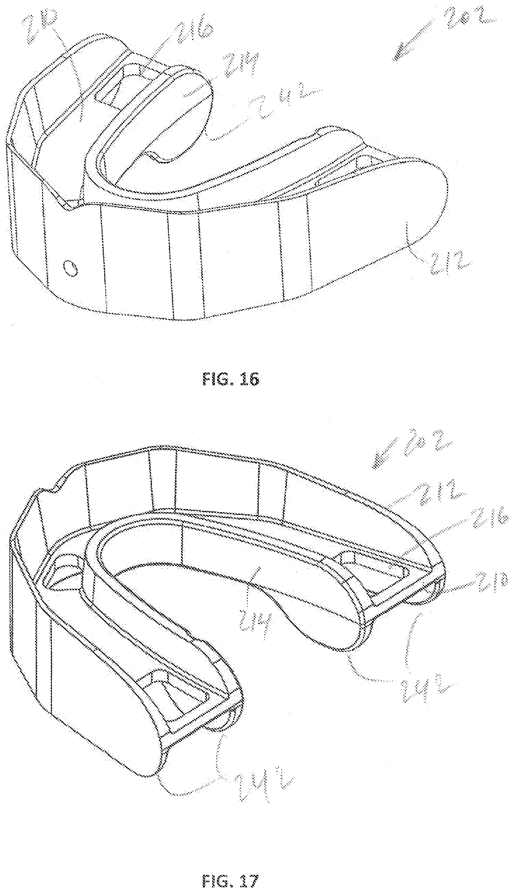

The lingual flange 114 of the base portion may extend upwardly from an inside edge of the bite platform or shelf 110. The lingual flange 114 may be adapted to hold the oral appliance in place laterally by way of interaction with an inside surface of the teeth and any dentition material 108 arranged therebetween. As shown, the lingual flange 114 may have a generally constant height above the bite platform 110 as it extends along the inside edge of the bite platform 110 and may include a radiused or curved rear edge. In one or more embodiments, the lingual flange 114 may be relatively short in height to avoid overly aggressive engagement with the roof of a user's mouth. In one or more embodiments, the height of the lingual flange (measured from bottom of tray to top of lingual flange) may range from approximately 5 mm to 10 mm, or approximately 6 mm to 9 mm, or approximately 7 mm to 7.5. The lingual flange may also have a thickness ranging from approximately 0.5 mm to 4 mm, or approximately 1 mm to 3 mm, or approximately 2 mm to 2.5 mm. While not shown, in one or more embodiments, the lingual flange 114 may extend below the bite platform for all or a portion of the length of the inside edge and thereby may engage the inside surface of the lower teeth and/or any dentition material 108 arranged therebetween. One example of a lingual flange extending below the tray is shown in FIGS. 16 and 17.

The labial flange 112 may be arranged opposite the lingual flange 114 and may extend upwardly from an outside edge of the bite platform 110. The labial flange 112 may be adapted to hold the oral appliance 100 in place laterally by way of interaction with an outside surface of the teeth and any dentition material 108 arranged therebetween. As shown, the labial flange 112 may have a generally constant height above the bite platform, but may include a slight taper as the labial flange extends toward the rear of the mouthguard. Like the lingual flange 114, the labial flange 112 may include a radiused or curved rear edge that returns down to the bite platform 110. The labial flange 112 may have a relatively larger height than the lingual flange 114 and may range from approximately 10 mm to 20 mm, or approximately 12 mm to 15 mm, or approximately 13 mm to 14 mm. The labial flange may also have a thickness ranging from approximately 1 mm to 7 mm, or approximately 2 mm to 6 mm, or approximately 3 mm to 5 mm. While not shown, in one or more embodiments, the labial flange 112 may extend below the bite platform for all or a portion of the length of the inside edge and thereby may engage the outside surface of the lower teeth and/or any dentition material arranged therebetween. One example of a labial flange extending below the tray is shown in FIGS. 16 and 17.

The labial flange 112 may also be adapted to receive and protect circuitry. As shown, for example, the labial flange 112 may include a series of component recesses 118 extending into an outer surface thereof. The component recesses 118 may be particularly arranged, sized, and shaped to receive components of the circuitry 104 described in more detail below. As shown in FIG. 4, for example, a rear portion of the labial flange 112 may include a component recess 118 adapted to receive and hold a corresponding portion of the circuitry. The component recess 118 may be sized and shaped to receive the corresponding circuitry portion where, when the circuitry portion is placed in the component recess 118, a back side of the circuitry portion is substantially flush with or slightly raised from an outside surface of the labial flange 112 and a snug and/or force fit is created around the perimeter of the circuitry portion. As shown, an alignment lug, tab, or pin 120 may be provided in the component recess or adjacent the recess for engaging a hole or slot on the circuitry 104 to assist with proper alignment of the circuitry relative to the base portion 102 and the respective recesses. As shown, a plurality of component recesses 118 may be provided on the base portion that correspond to respective portions of the circuitry. For example, a processor recess may correspond to a processor portion of the circuitry and a battery or other power source recess may correspond to a battery or other power source portion of the circuitry. A storage recess may correspond to a storage portion of the circuitry and so on. Various arrangements of circuitry components and corresponding recesses may be provided and the present application shall not be limited to the particular arrangement shown. Moreover, and as discussed in more detail below, some components of the circuitry may face outwardly rather than inwardly and, as such, a recess on the labial flange 112 may be omitted for such components. Between the component recesses, circuitry standoffs 122 may be provided. The standoffs 122 may be recessed from the surface of the labial flange 112, but may have a depth that extends into the labial flange 112 a shorter distance than the component recesses 118. The standoffs 122 may be adapted to engage flexible portions of the circuitry 104 and assist with holding the circuitry 104 in a suitable relative position relative to the labial flange 112 and the bottom of its recesses 118. For example, the standoffs 122 may be adapted to hold the circuitry 104 in a position relative to the labial flange 112 where the components of the circuitry in the recesses just touch the bottoms of the recesses and/or where the components of the circuitry have a slight gap between the components and the bottoms of the recesses 118.

The base portion 102 including the bite platform 110 and the labial and lingual flanges 112/114 may be made from a relatively soft plastics that are rigid enough to resist excessive twisting, form a proper dental tray during a boil/bite process, but are sufficiently soft to allow for some play or give around embedded electronics so as to avoid damage to the electronics if some twisting does occur. In one or more embodiments, the base portion 102 may include polyurethane, such as pellethane, polypropylene, polyethelene, ethylene vinyl acetate (EVA), or another elastomeric injection moldable plastic. In one or more embodiments, the base portion 102 may be a substantially monolithic element that is formed in a single injection molded process, 3D print process, machining process, or other manufacturing process. That is, the base portion may be substantially uniform in chemical construction and molecular structure throughout and may not include seams or other evidence of molecular fusions, for example.

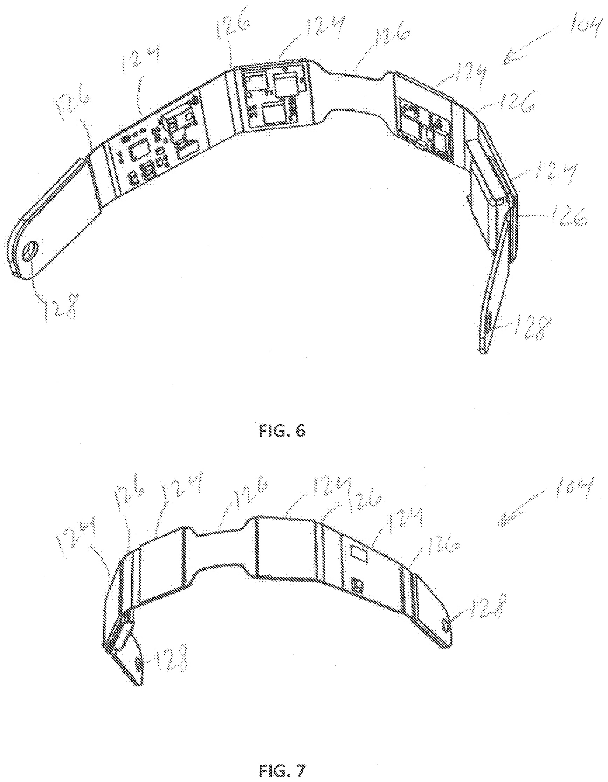

Turning now to FIGS. 6 and 7, circuitry 104 is shown. The circuitry 104 shown may be adapted to sense motion of the mouthguard and, thus, the user wearing the mouthguard. The circuitry may be further adapted to analyze the motion to determine results relating to the impact to the user and the potential for injury. Still further, the circuitry may be adapted to communicate the data and/or the results for further processing, display, analysis, or other use. In one or more embodiments, the circuitry 104 may include one or more circuits for measuring impact, tracking activity (e.g. step counting, activity awareness, positional dead-reckoning), biometric monitoring (e.g. heart rate, body core temperature, respiration rate, blood pressure, blood oxygen saturation, and others), location determination (e.g. GPS position or triangulation position within a defined environment based on known location beacons), and other functions.

The circuitry 104 may include a plurality of components pursuant to that functionality including a power source, one or more sensors, a computer readable storage medium, a processor, indicator lights, and electrical leads connecting the several components. As shown, the circuitry 104 may include one or more substantially rigid boards 124 and one or more substantially flexible links 126. In one or more embodiments, the circuitry 104 may include multiple, separate sub-circuits and/or components (such as a battery or sensor) connected by flexible circuit boards and/or insulated wires.

The rigid boards 124 may be provided at the computing component locations and may be relatively rigid and strong portions intended to protect the computing components from damaging forces such as lateral biting, for example. The rigid boards may also function to assist with proper positioning and securing of the sensors. For example, the rigid nature of the board together with epoxied securing of the boards (or other potting techniques) and including locating the sensor on particular portions of the board may provide for more suitable sensor results. With respect to locations of the sensors, locating sensing components such as accelerometers and/or gyroscopes or other microelectromechanical systems (MEMS) at particular locations on the boards may help to provide for a less noisy signal. For example, locating the MEMS devices at the corners of the boards may help to reduce or avoid noise in the signal. Still further, in one or more embodiments, one or more accelerometers and/or gyroscopes may be positioned along a centerline of a mouth. That is, these sensors may be positioned within the labial flange at or near the front of the mouthguard, for example. This may assist with avoiding issues with size and shape of a mouth when translating the sensor results to a center of gravity of a head, for example, since the centerline of the mouth may be assumed to be in line with the center of gravity of the head. In one or more embodiments, the rigid portions 124 may be a glass-reinforced epoxy laminate such as FR4 or a ceramic material may be used.

The flexible links 126 may be provided between the rigid boards 124 to allow the circuitry 104, as a whole, to move and flex with the mouthguard or other oral appliance. The flexible links 126 may include less rigid portions having wiring, leads, or other relatively flexible aspects of the circuitry arranged thereon and extending thereacross from the components on a rigid board 124 to components on a neighboring or more distant board 124. The flexible links 126 may be a copper or polyimide material, for example. In one or more embodiments, the thickness of the polyimide may be selected to avoid ripping or tearing when the mouthguard is twisted, for example. In some embodiments, the polyimide layer or layers may have individual thickness ranging from approximately 5 µm to 100 µm, or from 10 µm to 50 µm, or from 20 µm to 30 µm. These thicknesses may be sufficiently thick to resist ripping, but thin enough to provide flexible relief to the circuitry. The overall thickness of the flexible links (typically including polyimide layer(s), copper layer(s) for signals/ connections, adhesive layer(s), copper plating, soldermask, and/or other layer(s)) may range from 20 µm to 1000 µm, or from 50 µm to 700 µm, or from 100 µm to 500 µm. The wiring, leads, or other relatively flexible aspects of the circuitry 104 may be arranged on the flexible links as insulated wires or as insulated wires suspended within a tube, or as insulated wires that follow a serpentine pattern in order to allow slack for extending and retracting and avoiding stretching or resisting twisting forces when the mouthguard is twisted, warped, or similarly manipulated.

As shown, many of the components of the circuitry may be arranged on an inside surface of the rigid boards 124 such that when the circuitry 104 is placed against the base portion 102, the components may be arranged in respective recesses 118 of the base portion 102 and may be covered on an outside surface by the rigid boards 124. As shown, the circuitry 104 may include one or more alignment features 128 such as a hole or opening adapted to engage a protruding aligning feature 120 on the base portion 102. While the base portion has been shown to include protruding alignment features 120 to engage openings 128 on the circuitry 104, the opposite may be provided where the base portion 102 has an opening and the circuitry has a protruding element. As shown, the flexible links 126 may be sized and shaped to engage the standoff portions 122 of the base portion 102 and provide for proper or suitable relative positions of the components of the circuitry relative to the recesses 118 in the base portion 102.

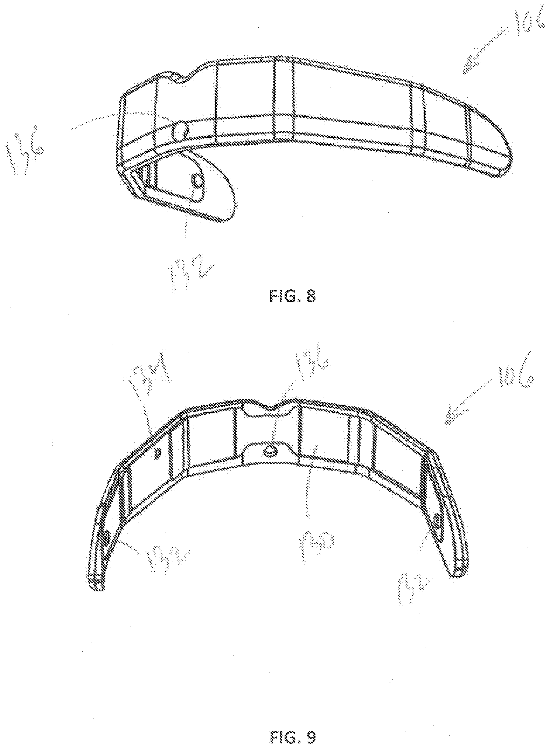

FIGS. 8 and 9 show a cover portion 106. The cover portion 106 may be adapted to cover the circuitry arranged on the base portion 102 and seal around the circuitry 104 by engaging a peripheral portion of the labial flange 112 of the base portion 102. The cover portion 106 may, thus, be sized and shaped to have a same or similar peripheral edge creating a cover portion having a size and shape similar to that of the labial flange 112 of the base portion 102. As shown, and where the circuitry is slightly raised above the outside surface of the labial flange 112 when placed thereon, the cover portion 106 may include a recess pattern 130 on an inside surface thereof to accommodate the raised portion of the circuitry 104. This may help to provide a more secure connection between the cover portion 106, the circuitry 104 and the base portion 102 by resisting lateral sliding of the cover portion 106 in an upward or downward direction relative to the circuitry 104 and the base portion 102. As shown, the cover portion 106 may include a nub or other protruding element 132 for aligning the cover portion 106 with the circuitry 104 and, thus, the base portion 102. In one or more embodiments, cover portion 106 may include an opening or window 134 arranged at a location corresponding to an indicator light or other viewing feature of the circuitry such that light, for example, may pass through the window 134 allowing users to see an indication. As also shown, the cover portion 106 may include a tether opening 136 for receiving a tether used to secure the mouthguard to a helmet, for example. While not mentioned above, the tether opening may be in alignment with a similar opening on the base portion allowing the tether to extend through the cover portion and into or to the base portion.

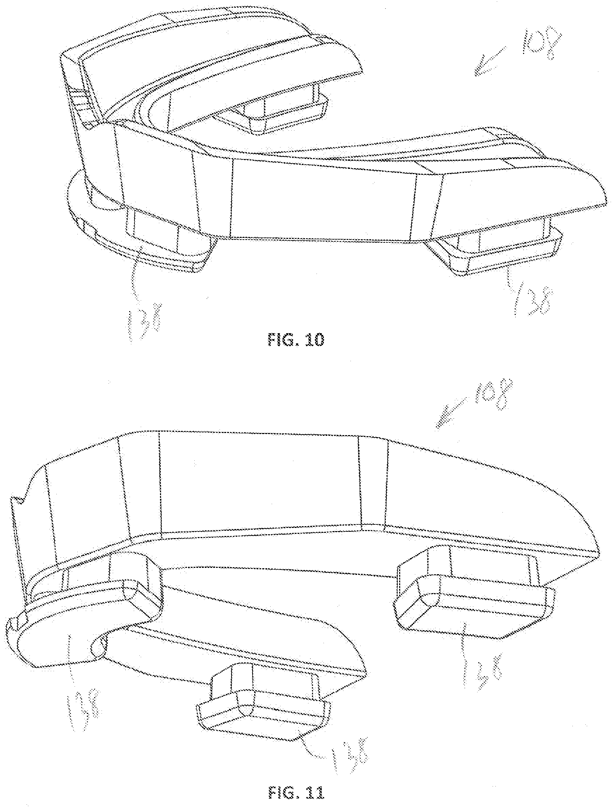

Referring finally to FIGS. 10 and 11, a dentition portion 108 is shown. The dentition portion 108 may be adapted to engage the teeth of the user and may be further adapted to allow for boil and bite formation. A large portion of the dentition portion 108 may be arranged within the flanges 112/114 of the base portion 102 and may have a plan view shape corresponding to the base portion 102 (i.e., u-shaped). Still further, when viewed in cross-section, the dentition portion 108 may have a substantially u-shaped cross-section for receiving the teeth of a user. The dentition portion 108 may also include front and rear pedestal portions 138 that extend through the passthrough openings 116 of the base portion 102 and have flared bottoms or flanges that spread out along a bottom surface of the base portion 102. These flared bottoms or flanges may be adapted to engage the bottom front and/or bottom rear teeth of the user and provide for a more secure position of the mouthguard within the user's mouth. The dentition portion 108 may be formed from a formable material such as ethylene vinyl acetate (EVA) or other formable material. That is, for example, EVA may be suitable for a boil and bite process allowing a user to customize a mouthguard to their own teeth by boiling the mouth guard and biting the mouthguard causing the EVA to reform to match their teeth shape.

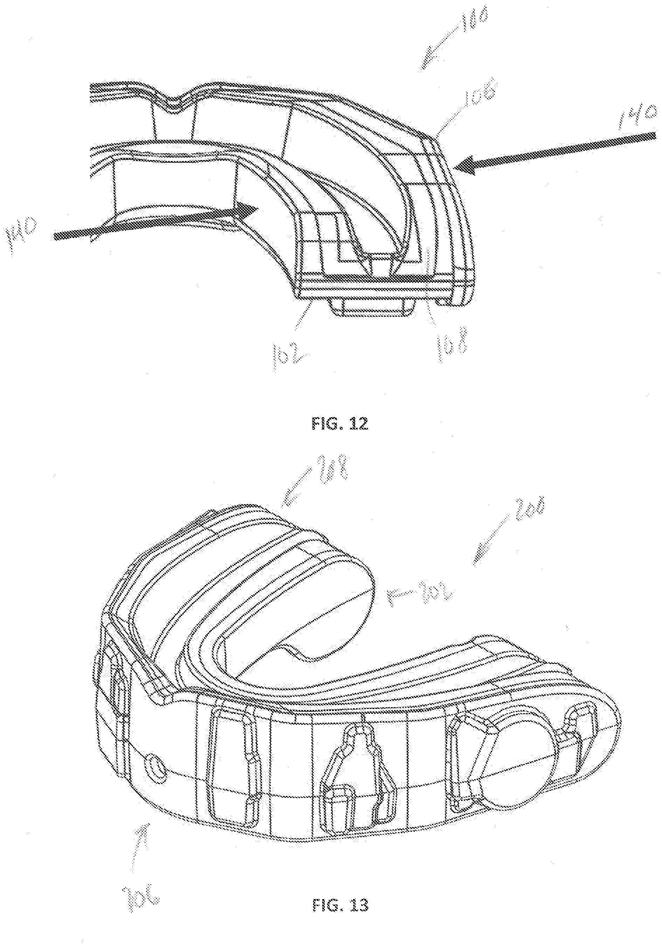

It is to be appreciated that while the present embodiment has been described as having circuitry 104 arranged on the labial side of the teeth (e.g., between the teeth and the cheek/lips), the circuitry 104 may also be arranged on the lingual side (e.g., between the teeth and the tongue). Moreover, while most of the components of the circuitry have be described as facing inward toward the teeth (e.g., on the teeth side of the supporting substrate), the opposite may also be true as is described in more detail with respect to FIGS. 13-23 below. So, while one option is to have the circuitry 104 facing the labial side of the teeth, another option is to have the circuitry facing the lingual side of the teeth. Moreover, if the circuitry is facing outward away from the teeth, it remains that the circuitry can be arranged on the labial or lingual side of the teeth. Still further, depending on the functionality desired, some components of the circuitry may be arranged facing outward while other components may be arranged facing inward. For example, and as discussed in more detail below, where sensors are used to sense whether the mouthguard is on the teeth, sensors facing inward may be used. Where sensors are used to sense ambient surrounding light, sensors facing outward may be used. Where an indicator light is used to notify a user, the light may face outward, for example. Dual facing components may be provided by providing components on both sides of a single rigid board or on multiple boards where one or more boards face a different direction. Still further, one or more components may be a free floating components. For example, batteries or sensors may be free floating. In the case of sensors, the sensor may be free floating if they are intended to sense in multiple directions. Other reasons for free floating components may relate to creating a comfortable fit, for example. Still other bases for providing free floating components may be realized.

With reference to FIG. 12, circuitry facing inward toward the teeth (whether on the labial or lingual side) may better protect the circuitry against lateral biting. That is, mouthguard users often insert the mouthguard ends in their mouth and chew on the cross section. The cross section of the mouthguard effectively forms an I-beam or a C-channel as can be seen in Figure 12 and the forces 140 from lateral biting are shown acting on the C-channel. If the components face outward (e.g., on either the labial or lingual side of the teeth), they will effectively be on top (or close to the surface) of this C-Channel profile when the user chews the cross section. Due to its shape, the C-Channel profile may be relatively stiff, even if the material it is made from is somewhat soft. Placing the components facing outward will also place them on top of the circuit board, which may also be somewhat stiff. Therefore, if circuit components are placed facing outward, the user may chew the cross section, crushing the components against the stiff circuit board and the stiff mouthguard cross section. Apart from chewing, any other impacts or forces on the outside of the mouthguard due to athletic hits, mouthguard dropping, or the like will also serve to crush the components against the stiff circuit board and the stiff mouthguard cross section. In contrast, if the components of the circuitry face inward toward the teeth, mouthguard durability advantages may be realized because the components may be protected on their back side by the rigid boards and on their front side by the opposing base flange and bite platform. That is, the rigid boards and/or opposing base flange and bite platform may resist bending, that may pop the components off their soldered pads, and may also spread out the force of the teeth over a larger area. Biting down on components with sharp teeth can concentrate forces and crack the components, especially ceramic components. Placing the components on the inward-facing side of the circuit substrate may reduce this effect by spreading out the biting force. Still further, with the components facing inward, the circuit board may include optical sensors that can sense tissue or physical objects within the channel of the mouth guard as discussed in more detail below. Particularly, proximity sensors may be used to identify when teeth are in the mouthguard and the mouthguard is, thus, in place in a user's mouth.

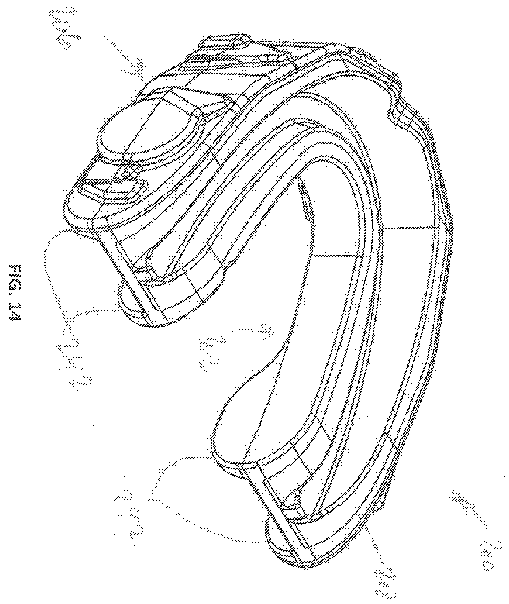

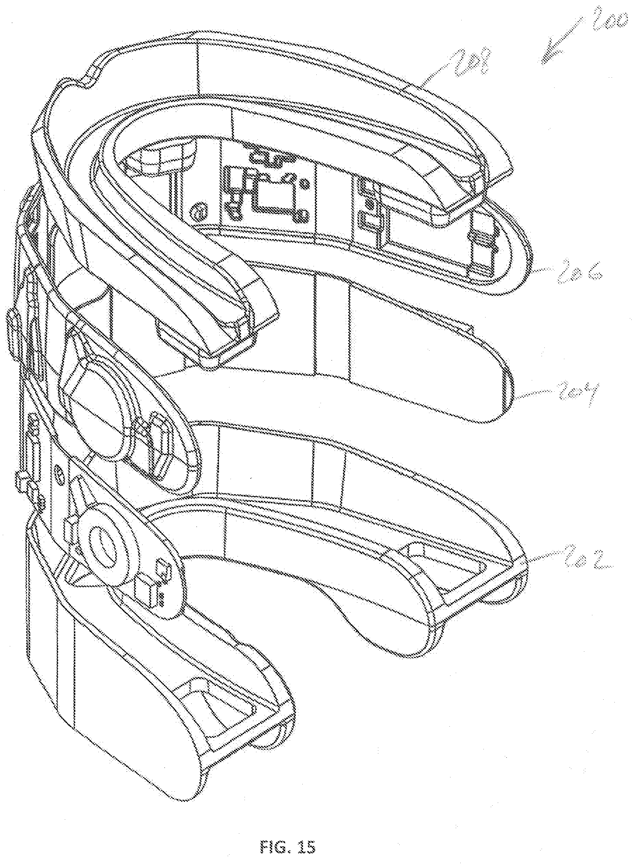

As mentioned, and while the circuitry 104 may be more vulnerable, there may be reason to place some or all of the circuitry facing outward away from the teeth. FIGS. 13-23 show an embodiment of an oral appliance 200 having at least some, if not all, circuitry 204 arranged on a labial side of the teeth and facing outward away from the teeth. FIGS. 13 and 14 show perspective views of the oral appliance 200, while FIG. 15 shows an exploded view thereof. As shown, the oral appliance may include a base portion 202, circuitry 204, a cover portion 206, and a dentition portion 208. FIGS. 16 and 17 show the base portion 202. The base portion shown may include the bite platform, tray, or shelf 210 similar to the base portion of FIGS. 4 and 5 and including the dentition portion flow through openings 216. The base portion 202 may also include a labial flange 212 and a lingual flange 214. In this embodiment, the labial and lingual flanges 212/214 may have one or more rudder portions 242 that extend below the tray. As shown, the base portion may not include pockets because the circuitry may face outward away from the teeth. It is to be appreciated that where one or more components of circuitry faced inward, pockets may be provided therefore.

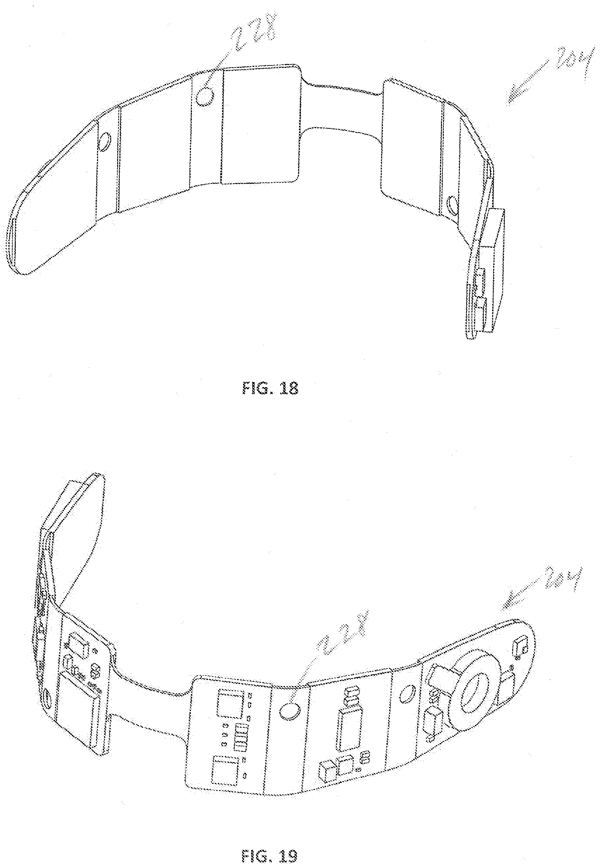

FIGS. 18 and 19 show circuitry 204 similar to the circuitry of FIGS. 6 and 7, but with components on an outside surface thereof. It is to be appreciated that some portions of the components may be placed on the inside surface. Moreover, alignment features 228 are shown for aligning the circuitry 204 with the base portion 202 and/or the cover portion 206.

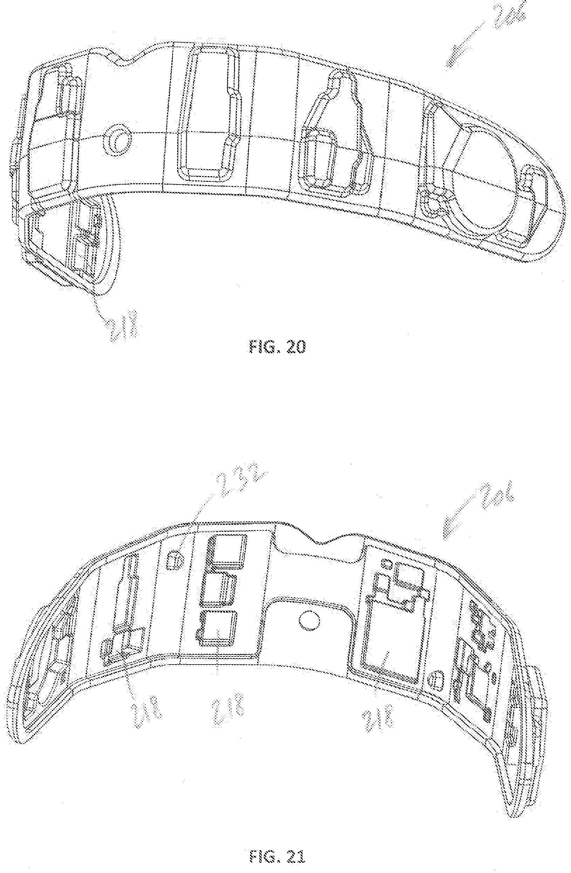

Turning now to FIGS. 20 and 21, a cover portion 206 is shown. In this embodiment, the cover portion 206 includes a plurality of pockets 218 adapted to accommodate the components of the circuitry 204. Corresponding alignment features 232 may also be provided.

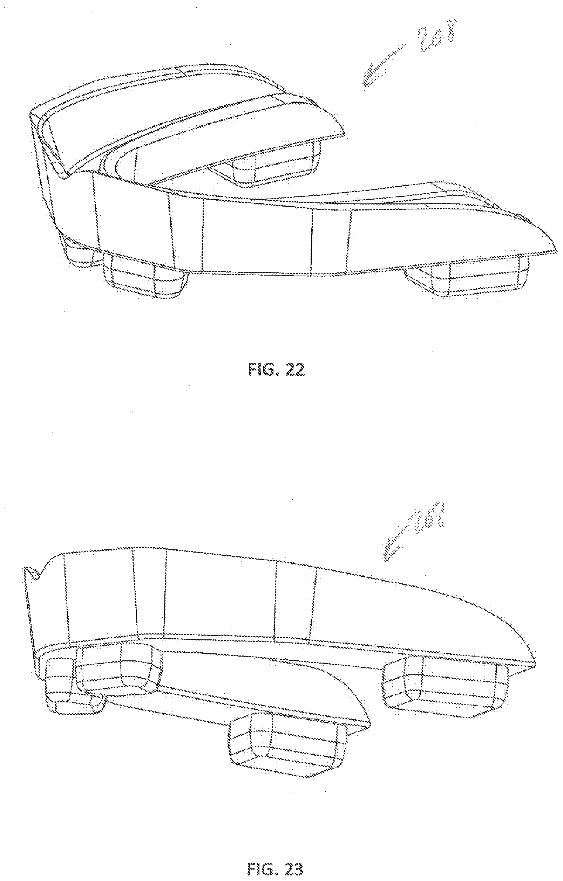

A dentition portion 208 is shown in FIGS. 22 and 23 that is similar to that shown in FIGS. 10 and 11.

In some situations, there may be a desire to determine if the oral appliance 100/200 is being worn by a user, as opposed to being in their hand or elsewhere. Knowing that a user is wearing a mouthguard may be helpful, for example, to improve reliability of measurements reported by the mouthguard based on knowledge that the mouthguard was actually in place at the time the readings were sensed. That is, knowing that the mouthguard is or is not in the user's mouth allows differentiation of false positive readings (created, for example, by the mouthguard hitting the floor after being dropped), from true head impacts where the mouthguard is in the user's mouth and/or is in place on the teeth.

In one or more embodiments, the circuitry 104/204 may be equipped with in-the-mouth (ITM) or on-teeth (OT) sensors to determine if the mouthguard is in a user's mouth or properly seated on the user's teeth. The sensors may come in one more forms including temperature sensors to detect the temperature increase from being placed in the user's mouth, proximity sensors to detect when teeth, cheeks, or lips are nearby, light sensors to detect when the mouthguard is in the dark mouth, humidity or liquid sensors, gas sensors, pressure sensors, or mechanical switches that detect the insertion of a tooth into the dentition area, and others. In each case, care should be taken to leverage the sensor technology (e.g., either ITM or OT) to reduce or minimize false triggers.

Proximity sensors may include light-based sensors that emit a light source (e.g., infrared) and detect its reflection, sensors that detect an electric field or a disturbance of an electric field, and capacitive sensors that can detect the capacitance of a user's finger, cheek, lips, teeth, etc. Proximity sensors used to detect the cheek or lips may be directed outward away from the teeth and proximity sensors used to detect the teeth may be directed inward. Facing the sensors inward may be advantageous due to the lesser chance that obstructions or other objects may trigger the sensors because the relatively narrow trough or channel along the mouthguard may not be as readily accessible as an outside surface of the mouthguard. For example, fingers or other potential obstructions are not very likely to fit into the narrow channel provided for receiving the user's teeth and, thus, may be less likely to falsely trigger the inwardly directed proximity sensor.

Of the several types of proximity sensors mentioned, capacitive sensors may be advantageous by being less sensitive to contacts that are not full contacts. That is, for example, capacitive sensors may be less sensitive to fingers, teeth, etc. if there is an air gap between the sensor's electrode and the object (e.g. tooth/finger) to be sensed. Better results may be provided if the entire distance between the sensor electrode and object is filled with a non-conductive substance such as glass or plastic. Where the teeth have been precisely fit to the mouthguard through the fitting process (whether boil and bite or custom mouthguard manufacture), other objects not so well fitted may have a lower likelihood of triggering a capacitive sensor. For example, fingers may be less likely to achieve a fully conformal fit and, therefore, may be less likely to inadvertently trigger the sensor.

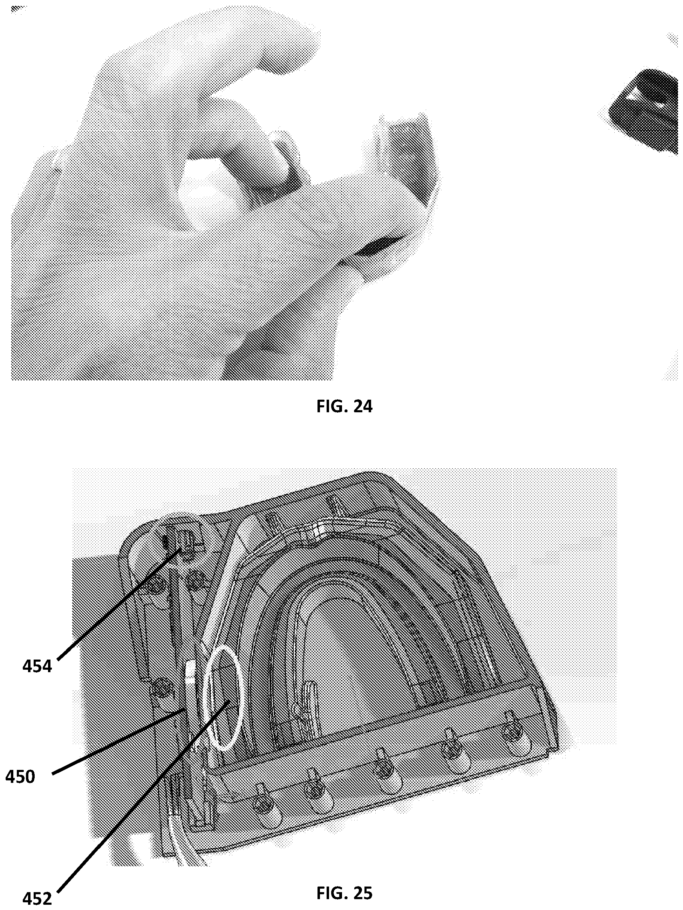

To further improve the low likelihood of sensors being triggered by fingers (e.g., whether light-based, electrical field based, or capacitive), the sensors may be placed in positions unlikely to be contacted by fingers simultaneously. For example, as mentioned, placing the sensors facing inward may limit false trigger situations to those where fingers are placed inside the channel for the teeth. This may be difficult due to the narrow nature of the channel. Additionally or alternatively, however, the sensors may be placed in locations that would make covering both sensors with fingers very awkward. For example, as shown in FIG. 24, a user may be forced to work very hard and/or consciously to trigger both sensors that are placed at different locations along the mouthguard tray. That is, where a sensor is placed near the rear portion of the guard and one is placed near the front of the guard, triggering both sensors may require that two fingers be placed in the tooth channel at different locations across the mouthguard, which may hardly, if ever occur when handling the mouthguard unless intentionally done. For this reason, the sensors would be less likely to be falsely triggered by fingers.

With reference again to capacitive sensors, such sensors sense objects that are arranged parallel to the electrodes much better than objects arranged perpendicular to the electrodes. By placing the capacitive sensor electrodes parallel to the teeth, the geometry of the mouthguard may cause the electrodes to be perpendicular to other potentially triggering objects such as tables, floors, palms of hands, or other objects on which the mouthguard may be commonly placed. As such, capacitive sensors may be less likely to be falsely triggered by objects that are set on top of the mouthguard or objects on which the mouthguard is set.

Still further, capacitive sensor electrodes may be designed with a strong preferential sensing direction (e.g. forward or backward) by shielding the opposite side with an electrical ground layer to prevent sensing capacitance of objects in that direction. In the case of capacitive electrodes pointing at the teeth, a ground layer may be placed opposite the teeth such that the capacitive sensing electrode is between the teeth and the ground layer. In this way, the capacitive sensing electrode may have a strong preference to sense in the direction of the teeth, and may be less likely to sense cheeks, fingers, and other objects on the opposite side of the mouthguard.

Considering the above, one or more embodiments of a mouthguard may include circuitry that includes multiple capacitive sensing electrodes facing toward the teeth in positions where the user's hand is unlikely to cover all of the electrodes simultaneously. The opposite side of the sensor facing the cheek may be covered by a ground layer to prevent sensing of the cheek or fingers from outside of the mouthguard.

The ITM/OT sensors may reliably be able to determine how well the mouthguard fits and is retained on the athlete's teeth. This may be determined via a combination of proximity sensor reading to determine how well the mouthguard is seated on the teeth, while assessing the quality of the waveform received via an impact on the accelerometer or gyroscope. That impact may be created by an actual impact to the head of the athlete, or via a haptic input created by the mouthguard circuit board itself. The ITM/OT sensors may also help determine whether this baseline has changed over time. For example, the data may be used to determine how long, on average, it takes for a boil-and-bite mouthguard to wear out, and thereby give a baseline for establishing product warranties, or whether a certain user needs a replacement, as their mouthguard is no longer coupling well enough to get high quality data.

In some cases, a calibration procedure may be used to determine if the mouthguard is on the teeth or not, determining a baseline reading of completely on the teeth and completely off, or whether there is an inconsistency in manufacturing (e.g. an air bubble in the plastic) that is blocking or refracting the sensor's light in an unexpected way, resulting in higher or lower sensor results than expected. A user interface application may allow users to notify when the mouthguard is known to be on the teeth and off the teeth, so that determinate readings can be made and individualized thresholds may be created for the various sensors determining whether the mouthguard is ITM or OT.

It is to be appreciated that while the ITM and OT sensors have been described as part of the circuitry of the present embodiment, such technology has value in several applications outside of the particular details of the present embodiment. As such, mouthguards without the particularly described base portion, circuitry, cover portion, or dentition may benefit from the described ITM and OT sensors. Such ITM and OT sensors may be present and/or provided with most any sensor capable oral appliance or other sensing device.

In one or more embodiments, the oral appliance may not be a molded mouthguard. Rather, with thin, flexible circuit such as polyimide or polyester, a thin assembly with a circuit may be created that adheres directly to the teeth using adhesive, for example. In such embodiments, the circuit may be disposable or have a disposable element and a separable reusable element. Still other types of oral appliances may be provided.

As mentioned, part of the difficulty of developing a smart mouthguard (e.g., one that can sense motion, analyze that motion, and/or communicate details about that motion) relates to the high temperatures and pressures associated with injection molding, which is a common mouthguard manufacturing technique. In one or more embodiments, a method of manufacturing may involve injection molding the mouthguard in a manner that allows the circuitry to be protected from, and/or for better management of, the temperatures and pressures of injection molding. Additionally or alternatively, other manufacturing techniques may be used that avoid the use of injection molding and, as such, avoid one or more of the concerns associated with injection molding. In either case, mouthguards may be manufactured on a large scale for multiple users while allowing for customization by the user after manufacturing. Alternatively, mouthguards may be manufactured on a custom basis where the mouthguard is manufactured to particular specifications (e.g., a particular dentition geometry) of the user. Still further, in one or more embodiments, mouthguards may be manufactured on a quasi-custom basis where a common general dentition geometry is used that meets or is consistent with a large number of members of a given population. For example, a high number of adult males may have common characteristics associated with their dentition geometry and a mouthguard meeting the specifications of those common characteristics may be manufactured and further customization that is less extensive may be performed.

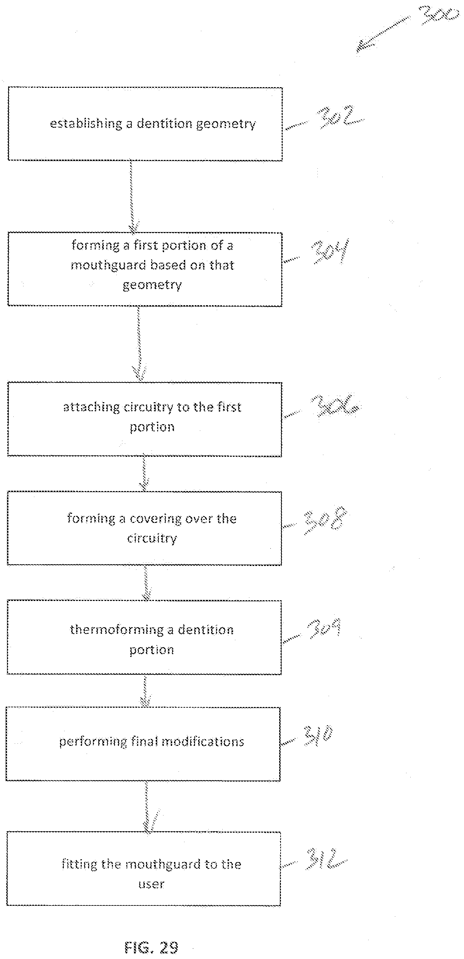

In one or more embodiments, as shown in FIG. 29, a method 300 for making a mouthguard may include establishing a dentition geometry (302), forming a base portion of a mouthguard based on that geometry (304), attaching circuitry to the base portion (306), forming a covering over the circuitry (308), thermoforming a dentition portion (309), performing final modifications (310), and fitting the mouthguard to the user (312). Some of all of these steps may be performed in a variety of ways.

Establishing a dentition geometry (302) may be performed with an eye toward creating a general applicability mouthguard with broad applicability and a relatively high amount of customization to be performed by the user. Alternatively, establishing a dentition geometry may be performed with an eye toward creating a customized mouthguard that is specific to a particular user's dentition geometry. Still further, establishing a dentition geometry may be performed with an eye toward creating a quasi-custom mouthguard that is based on dentition geometry characteristics of a set of users allowing a mouthguard to be created that has relatively broad applicability, but a relatively low amount of customization to be performed by the user.

With respect to general applicability mouthguards, establishing a dentition geometry (302) may include aggregating data relating to general mouth sizes and gum curvatures as well as general teeth sizes and shapes. Given this data, a series of mouthguard sizes may be established based on logical breaks in the data that establish envelopes covering a broad range of dentition geometries. Given these envelopes, a series of base portion sizes and shapes may be established to cover a broad range of dentition geometries. In this situation, any and all customization of the mouthguard may be performed by the user using a boil and bite process to conform the dentition portion of the mouthguard to fit their dentition geometry.

With respect to custom mouthguards, establishing a dentition geometry (302) may be performed in a couple of different ways. For example, the method may include 3D scanning of a user's mouth or taking a negative physical impression. The method may also include creating a positive physical impression based on the scan or the negative physical impression. This positive physical impression may be created in a plastic, stone, metal, or other material of sufficient strength and thermal resistance to withstand the anticipated pressure and heat of one or more thermoforming steps.

With respect to 3D scanning, a scanner such as a commercially available technology for form-fitting dental braces (e.g. Invisalign) may be used. A scanning probe may be inserted in the user's mouth, and the teeth and jaw scanned in three dimensions, creating a 3-dimensional computer model suitable for manipulation in CAD software. The computer model may be modified in this step to improve perceived comfort, enhance mechanical coupling of the sensors to the user's teeth, or to improve manufacturability of the custom mouthguard.

Based on the 3D scan of the subject's mouth and teeth, which may be a 3D scan of a negative impression, or a 3D scan of the teeth themselves, a high-resolution positive physical impression may be printed on a 3D printer such as a stereolithography (SLA) printer. This print may be a full-scale representation of the user's teeth and jaw area with adequate resolution to accurately represent the subject's unique mouth geometry. A physical copy of the 3D scan may also be created with other manufacturing methods such as milling. This model may be modified in a computerized fashion for better fit or to add features for later manufacturing, such as cutting/trimming guides, or localization features for circuit board placement.

With respect to quasi-custom mouthguards, establishing a dentition geometry (302) may be performed by 3D scanning, or taking negative physical impressions, of the mouths of a representative group of a larger group of users. Based on the dentition geometries captured, common characteristics across the representative group may be assumed to exist in the larger group of users. A quasi-custom dentition geometry may, thus, be established that accommodates the group of users and leaves only the finer details of their dentition geometry for customization. For example, a 3D models of the quasi-custom dentition geometry may be created using dentition geometries captured from the representative group of users and the quasi-custom dentition geometry may be 3D printed or milled, for example. This approach may allow for a smaller mouthguard having a smaller (e.g., narrower and shallower) channel because the later customization variations from the created mouthguard may be less drastic.

With the dentition geometry established, the method may include forming a first or base portion of a mouthguard based on that geometry (304). In one or more embodiments, forming a base portion of a mouthguard may include injection molding the base portion or 3D printing the base portion, for example. Where a customized mouthguard or a quasi-customized mouthguard is being manufactured, the injection mold may include an insert or other device that establishes the dentition geometry within the mold. That is, the insert may include the dentition geometry such that the top and bottom of the bite platform or tray and the inside surfaces of the flanges are molded to fit the desired dentition geometry. In one or more embodiments, when the base portion is being created for a custom or quasi-custom mouthguard, the base portion may be molded from EVA and the further formation of a dentition portion may be omitted. That is, the base portion may be formed to fulfill the roles of the base portion and the dentition portion. However, another approach would be to create a relatively generic base portion even in the case of a customized or quasi-customized mouthguard where the base portion is sized and shaped to receive a dentition portion. In this embodiment, the upper and lower surfaces of the bite platform or tray and the inside surfaces of the flanges may be relatively flat and/or may have a relatively generic surface profile for receiving the dentition portion. Where a general applicability mouthguard is being manufactured, the injection mold may be sized to create a selected size base portion, or inserts may be used to adjust the size of the mold for a particularly sized base portion. However, in the case of a general applicability mouthguard, the base portion may be relatively generic with respect to the surface profile of the bite platform or tray and the surface profile of the inner surfaces of the flanges.

More particularly with respect to the molding discussed, a first one or more layer(s) of dental material (e.g. EVA plastic) may be thermoformed over the 3D printed model to form the base layer for the mouthguard which may be the layer closest to the wearer's teeth, and a base layer(s) to which to adhere the circuit material. Due to the accurate 3D scan, this first layer(s) will fit the user's teeth with high comfort and precision. Thermoforming may be conducted with a small form factor dental thermoformer that applies a relatively low positive pressure to the outside of the model and plastic sheet, or thermoforming may be conducted with a larger vacuum pressure application that can apply higher negative pressure and can form many models at once. Alternatively, a thermoset plastic may be formed over the 3D printed model. A two-part material or other curable material may also be layered over the 3D printed model to form the first layer. Other layers may be added over this layer. These layers may be clear, or have clear sections to allow for optical sensors to have a proper window of view inward at the teeth. These layers may also be colored or have graphics.

Since injection molding pressures and temperatures may be higher than the circuitry is able to withstand directly, in one or more embodiments the first piece of the assembly may be a piece (e.g. a first molded part, or "first shot") that is molded alone without the circuitry present (e.g., the base portion 102 if circuitry facing inward; cover portion 106 if circuitry facing outward). In such embodiments, this first shot may have recesses designed into it to accommodate the circuit and its components. The first shot may include a perimeter outline (e.g., along a periphery of the labial flange; along the inner peripheral surface of the cover portion if the circuitry is facing outward) that allows a tight seal when injection molding behind the board, which may help to prevent molten plastic around the board to the component side. In this way, the circuitry may be placed into these first shot recesses, and this first shot may be sealed or adhered to the circuit.

In one or more embodiments, the pockets established in the 1st shot closely match the component shape and size, so that the force of injection molding during the 2nd shot, discussed below, is transmitted most into the base of the circuit board around the components, and any force directly against the component top is quickly supported by 1st shot plastic. Pockets in the first shot are intended to minimize the acceleration of the components from injection molding force before contacting the top of the pocket in the 1st shot.

In some embodiments, the recesses may be of a general, slightly oversized sort, which are meant to give space for components so that they do not move against the first shot, or if they are coated in an epoxy that may not be able to meet specific spacial tolerances. In this embodiment, the stiff section(s) of the PCB(s) may be thick enough to resist injection molding pressure, and the edges of the first shot may hold the PCB(s) up from the recesses.

The method may also include attaching the circuitry to the base portion (306). In one or more embodiments, attaching the circuit to the base portion may include adhering the circuitry to the base portion. In one or more embodiments the adhesive may include EVA and, in some cases, 100% EVA. Additionally or alternatively, an adhesive such as PSA may be used on the ends of the circuitry, which may have removable plastic coverings for ease of use. The plastic coverings may be removed when the circuitry is being attached to the base portion and the adhesive may help maintain the circuitry in proper position attached to the base portion until the cover mold can be performed. Still further, the first shot or base portion and circuitry may have mating features discussed above such as pins and holes, perimeter recesses, and the component recesses to align, register, and retain them together. In one or more embodiments, attaching the circuit may include aligning any and/or all alignment features when attaching the circuitry.

Depending on the anticipated performance of seal between the base portion and the cover portion discussed below, the circuitry may be sealed prior to, during, or after adhering the circuitry to the base portion. That is, sealing materials or conformal coatings may be placed over the circuit board before the outer layer of plastics is formed. This may provide a second layer of defense if the outer layer (e.g., cover portion, base portion, seal between base portion or cover portion) is punctured or otherwise breached.

The method may also include covering the circuitry with a cover portion (308). After the circuitry has been adhered to the base portion, an additional layer or layers may be placed over the top of the circuit to protect the circuit from damage due to impacts, compression, and/or water and moisture. These additional layers may be thermoformed, thermoset, two part cured, or other methods of deposition.

In one or more embodiments, forming the cover portion may include loading the first shot and circuit together into a second injection mold and injecting liquid plastic into this second mold to encapsulate the circuit between the first and "second shot" created by this second mold. The second shot may seal to both the first shot and the circuitry to prevent water ingress into the assembly. The first and second shot may form a hermetic seal around the circuit assembly. The arrangement of the circuitry with the components of the circuitry facing inward with a rigid board behind them may allow for component protection from the second shot injection molding pressure and temperature.

The 2nd shot overmold process design may protect the circuit from the molding process. Several components may be sensitive to the heat and pressure of injection molding. For example, Li-ion batteries, and, in particular, Li-poly batteries, may experience internal shorting and premature failure if exposed to temperatures greater than 60-100C, or pressures as high as those present in injection molding. Other components such as ceramic capacitors, resistors, and silicon ICs may be broken or their solder joints ripped off the circuit by the high pressure flowing plastic. For these reasons, in some embodiments, the components may face the pre-molded first shot, and the second shot injected liquid plastic may be directed at the back side of the circuit board. In this way, the circuit board and any associated rigid stiffener portions applied to it may serve as a mechanical barrier to the temperature and pressure of the second shot. The rigid stiffener and flexible circuit material may better distribute the pressure of the molten plastic injected in the second shot and it may thermally isolate the components from the molten injected plastic.

It is to be appreciated that the cover portion may be substantially fused to the base portion around the perimeter of the circuitry. The cover portion may form a cold joint with the base portion where the crystallization between the two portions is evident, but, nonetheless, fused. However, and depending on the temperatures used for thermoforming the cover portion, a more monolithic formation may be created between the cover portion and the base portion.

The method may also include thermoforming a dentition portion (309). That is, the support material of the mouthguard may help to maintain the structural integrity of the mouthguard even when boiled for fitting. However, the mouthguard when user customization is needed, the mouthguard may include a material that softens when boiled so that the user may bite down into it to form an impression of their teeth. Because of these two competing requirements, there may be a third shot of plastic that is a different composition than the first and second shots. The first and second shot may encapsulate and protect the circuit board between their two layers, and the third shot may be molded over the second shot to provide the softer material for the user to form their teeth impression in. The third shot material may be EVA, which softens in boiling water adequately to form an impression of the teeth. The third shot may not be well suited for providing structural support and the first and second shots may provide the structural support.

Accordingly, for example, where base portion was not originally manufactured with customized or quasi customized profiles, the base portion, circuitry, and cover portion may be placed in a mold and the dentition portion may be molded to generally fill the channel of the base portion between the flanges and on top of the bite platform or tray. The openings through the bite platform may allow dentition material to flow through the bite platform or tray to create dentition pads on a bottom side of the bite platform or tray. Depending on the type of mouthguard being manufactured (e.g., generally applicable, custom, or quasi custom), the mold may include dentition profile inserts to cause the dentition portion to reflect the desired dentition profile of a user or set of users as the case may be.

The method may also include making modifications to the molded or 3D printed mouthguard (310). For example, the method may include trimming, polishing, curing, and/or other post-processing techniques performed on the base portion, the cover portion, and/or the dentition portion.

The method may also include fitting the mouthguard to the user (312). The smart mouthguard may be fit to the user when complete. This may be the first time the user is wearing the mouthguard, since the design was based on scans and 3D prints. Because of this, the fit may be adjusted at this point for comfort. In some cases, the mouthguard may be boiled (with the circuit now inside) to soften it. The user may then bite down on the mouthguard to better conform it to their mouth. Trimming and polishing steps may also help improve fit and comfort for the user.

More particularly, the mouthguard may be cut, trimmed, or reduced in thickness in a fashion that minimizes material on the upper gums and/or lingual area. The mouthguard may be cut, trimmed, or reduced in thickness between front incisors and lips while maintaining a proper bridge to function as a mouthguard. These are areas that may be most sensitive to user comfort, but do not have as much of an effect on mouthguard retention. Fillets and rounds on all edges of the mouthguard design may be helpful to improve user comfort and to alleviate lip damage in the event of a direct impact. A removable or permanent lip guard outside the mouth may also be attached and may house additional electronics or antennas to improve the range of data transmission. A removable or permanently attached tether may be attached to the boil and bite mouthguard or custom mouthguard. This tether may include an antenna to improve the range of data transmission. If the tether is removable, metal contacts leading to the circuitry may be sealed in molding or printing process to protect the circuitry from water or moisture infiltration.

While the above discussion has focused on thermoforming and/or injection molding, oral appliances may alternatively or additionally be 3D printed around the circuit material. Other additive manufacturing methods may also be used. 3D layers may be printed to precisely match a specific user's mouth and teeth based on a 3D scan, as described above. Alternatively, a more general applicability mouthguard may be printed. When the base portion is printed, the printing may be paused to insert the circuit. The printing process may continue to encapsulate and seal the circuit in the 3D printed or additive manufactured smart mouthguard. The printed mouthguard may use one or many materials to achieve a comfortable, good fit, good retention, good protection of the circuit in operation, and other desirable mechanical parameters.

While the method 300 above has described a first shot and a second shot for sealing the circuitry, one or more embodiments, may include a single shot that wraps around the circuit in its entirety. Moreover, even with the two shot approach, molding temperatures and pressures may be helpful to monitor and control injection temperatures and pressures. Accordingly, strategies may be used to control the pressures and temperatures the circuit is subjected to such as using lower temperature molding processes or secondary operations to complete the enclosure, or other strategies. In particular, the number and location of the injection molding gates may be designed in a way that reduces the overall pressure of injection molding on the circuit by increase the number of gates. Additionally or alternatively, gates may be strategically arranged to move localized pressure and heat from individual gates away from especially sensitive components. Gates may also be located at areas that ensure the board is forced properly into its recess when injection first begins, and that the board is not moved or lifted when injection pressures are first applied, which would allow molten plastic into areas it is not intended. Generally, this means that injection must first begin at the beginning of the board and work outwards, which may be achieved through gate location and runner length definition (slightly longer runners for each gate outward from the center).

In some embodiments, the circuit is one-sided, and the inert carrier side (e.g. the circuit board itself, which may be made of substrates such as FR4, polyimide, ceramic, or other materials) is the only side subjected to the temperatures and pressures of the molten second shot plastic. In some embodiments, the pressure of the second shot molten plastic entering the mold pushes the circuit substrate tightly against the finished first shot plastic part, effectively sealing the two together against the second shot molten plastic entering between them.

Despite efforts to protect the circuit from the molding process with molding design, the circuit may still experience stresses due to the molding process and due to normal in-mouth use (e.g. chewing, bending, and the like). In some embodiments, the circuit and components may be encapsulated in a material such as epoxy to protect it against molding and normal use. The encapsulant may serve to better adhere components to the circuit board, potentially ensuring that the electrical solder joints do not experience significant mechanical loading. The encapsulant may be better suited to withstand mechanical stresses than the solder joints. The encapsulant may serve to protect components from movement against the first shot as the mouthguard is twisted.

In one or more embodiments, the oral appliance may include multiple potential failure modes. For this reason, the oral appliance may run automated diagnostic self-tests. These self-tests may be run on a regular basis, for example when the mouthguard is removed from the charger, when a specific time has elapsed since the last automated self-test, or at a specific time each day. The automated self-test may run diagnostic tests on things such as the battery state of charge, the sensors in the mouthguard, the wireless connection, the memory, or control circuits. The results of the automated self-test may be communicated via flashing light, audible sound, or vibration of the smart mouthguard itself, via a status message sent to the receiving device (e.g. a tablet computer), or logged to an internet server for real time reporting or assembly of summary reports of the self-test status of one or more mouthguards.

The self-test may test the function of such things as motion sensors, including accelerometers and/or gyroscopes for impact and/or motion monitoring. The tests may include a communication test to ensure that the motion sensor(s) or other critical components or systems are present, powered on, and communicating correctly. The tests may also include a quality test on data from the motion sensor. In some cases, a small amount of noise is expected from the sensor. The mouthguard may read data from the motion sensors and ensure there is some noise, which may be expected. In some cases, the motion sensor may have a built-in self-test that the mouthguard may execute.

There are several different demands on a power source for a smart oral appliance. In one or more embodiments, particular demands may be met and traded off against other demands in an attempt to achieve the desired mix of performance. The most straightforward tradeoff of power source demands for an oral appliance may be between the useful battery life and the size and weight of the oral appliance. The power source may include a chemical battery, such as a Li-ion, Li-poly, Solid state Li-ion, Nickel Metal Hydride, Lithium Iron Phosphate, primary Lithium, or Alkaline battery. In one or more embodiments, a rechargeable battery may be provided to allow the oral appliance to be recharged and reused throughout the season. The Lithium family of batteries generally provide the greatest energy density of batteries on the market today, so they may be a reasonable choice for an oral appliance. However, other battery types including types not listed may be provided.

The power source may be adapted to withstand pressure applied somewhat uniformly across the battery, such as during injection molding, or to withstand pressure applied over a smaller area, such as due to a bite down force from the user. For example, batteries with a relatively rigid metal enclosure such as a Li-ion, NiMH, primary, Lithium, or Alkaline may be used. A solid state battery may also be used for purposes of resilience under pressure, because its solid electrolyte makes it less likely that the pressure will damage the battery's internal layers. A Li-poly battery may have the highest energy density, and a thin streamlined package and, as such, may provide a strong tradeoff between battery size and capacity and resilience under pressure. In some embodiments, a battery that does not usually withstand injection molding pressure well (Li-ion, etc.) may be protected by a pre-molding process such as encapsulation in epoxy, low-pressure molding in a protective sub-enclosure, and/or other process(es) to shield it from injection molding pressure.

In addition to pressures, the battery may be adapted to withstand high temperatures, such as during boiling or during injection molding. In some embodiments, the temperature of the battery may increase to up to 100 °C during the boiling or molding processes. In such cases, a solid electrolyte battery may be well suited to the temperature requirements. Solid electrolyte batteries may survive temperatures of over 150 °C without significant degradation.

In some embodiments, a capacitor such as a super capacitor may be used as an energy source. The energy of a capacitor in Watt-hours is equal to ½^{∗}C^{∗}(V22-V12)/3600, where C is the capacitance in Farads, V2 is the starting (charged) voltage of the capacitor, and V1 is the ending (discharged) voltage of the capacitor. To be equal to a 35 mWh battery, for example, the capacitance would need to be 16 Farads.

In some embodiments, a power source may be a non-rechargeable battery, cell or power source, and the mouthguard will be used until power is consumed and then disposed. This would be the case with a coin cell or button cell type battery.

In some embodiments, energy harvesting may be used to supplement or replace a battery power source. An energy harvester may be more able to withstand temperatures and pressures of the mouthguard manufacturing process. Energy harvesters may convert energy such as thermal, activity, RF, or light into electrical energy to power the mouthguard or to recharge the mouthguard's other power source(s).

In some embodiments, a thermal harvester or thermoelectric generator such as a thermocouple, thermopile, Peltier diode or a Seebeck generator may be used to convert temperature differentials into usable electric energy. In some embodiments, the temperature differential between the wearer's mouth and an ambient temperature outside the mouth may be used to generate electrical power. In some embodiments, the smart mouthguard may have a portion that protrudes from the mouth in order to be exposed to an ambient temperature outside the mouth. For example, a tether or other protrusion may be provided.

In some embodiments, an activity harvester may be used as a power source. Such an activity harvester may convert the motion of the mouthguard in the wearer's mouth into usable electric energy. The activity harvester may be vibrational in nature, for example, by employing a resonant beam structure. The activity harvester may be rotational, for example, by using a spinning component such as a counterweight on a shaft such as with a self-winding watch coupled to a small rotational generator.

In some embodiments, a solar harvester may be used as a power source. Such a harvester could convert ambient light into usable electric energy. In some embodiments, the smart mouthguard may have a portion that protrudes from the mouth in order to be exposed to ambient light for charging. For example, a tether or other protrusion may be provided.

Since the mouthguard must fit into a mouth, strong consideration may be given to size to ensure comfort of the mouthguard in the mouth. An excessively large mouthguard may be uncomfortable to many users. The size limitations of a smart mouthguard may restrict the size of the power source. In such cases, the power source may be moved outside of the mouthguard. In some embodiments, the power source may be a battery that is attached to the mouthguard but is allowed to protrude outside the mouth or to be attached to a portion of the mouthguard that is outside the mouth.

In some embodiments, the mouthguard may harvest RF energy from an RF emitter outside of the mouth, converting this RF energy into useful electrical energy. In some embodiments, the RF emitter may be worn elsewhere on the wearer's body, such as inside of a helmet or on a shoulder near the mouth. In some embodiments, there may be one RF emitter per smart mouthguard-equipped player. In some embodiments, there may be fewer RF emitters than smart-mouthguard-equipped players. In some embodiments another person on the field of play such as the official may wear an RF emitter to power mouthguards on the field. In some embodiments, an RF emitter may be on the sidelines of the field and power the mouthguards on the field.

In some embodiments, the mouthguard may be charged wirelessly, using inductive or resonant charging. In some embodiments, the wireless charging may be accomplished with near field inductively-coupled charging. In this embodiment, as shown in FIG. 25, a wireless transmitter coil 450 may be placed in a case for the mouthguard in a place where a receiving coil 452 on the mouthguard may align with this transmitter coil. This may result in each mouthguard having its own charging coil circuit enclosed in an Individual Charging Case (ICC) or strap charger placed over the receiver coil on the mouthguard. The transmitter coil may be modulated at a frequency that transfers power to the receiver coil on the mouthguard. This transmitted power may be rectified and/or conditioned (with, for example, a rectifier, voltage multiplier, low pass filter, and/or other methods) to provide the power to charge the power source (e.g. battery or other technology mentioned above) on the mouthguard. The receiving coil in the mouthguard may be a physical component with coils of wire, or may be embedded in the layers of the mouthguard circuit, in its traces. A sheet of ferrite backing material may be used to reflect the transmitted magnetic field and thereby raise its transmission efficiency.

In some embodiments, the wireless charging may be accomplished with mid field wireless charging. In this embodiment, there may not need to be a 1:1 charger:mouthguard ratio. A charging case may hold one or more mid field transmitters that can each charge one or many mouthguards, as long as they are within the charging envelope of the transmitter. This approach relaxes the focus on precise coupling of transmitter and receiver coils.

In some embodiments, one or more far field transmitters may be used, in the team case or separately. They may use mesh-network coverage to link together and cover larger spaces. This may allow devices to be charged even while in use, which allows a smaller battery or other power technology mentioned above, for greater comfort of the user.

In some embodiments, the ICC may be powered by a power supply adapter or by USB power. The ICC may be plugged into a USB cable via a connector such as a USB Micro B or C connector 454. Figure 25 shows a micro USB connector circled in orange that is attached to the charging transmitter circuit board. As shown, the ICC may include a transmitter and a receiver coil embedded within the mouthguard.

Wireless charging may be sensitive to the relative orientation and location of the transmitter and receiver. As such, the ICC may have features to assist with suitable position of the mouthguard so that the coils (Tx and Rx) are properly aligned with each other. There may also be an indicator (LED(s), LCD screen, audible buzzer, Bluetooth or other signal, and/or other method(s)) to notify the user that the mouthguard is not present, not properly aligned, or of its general status.

The power connector of the ICC may be connected to a power supply such as main power or a battery bank using a power cable such as a USB cable. In some embodiments, the power cable may be a USB A to USB micro B cable, where the micro B side plugs into the charging case. Other connector that may be used are other standard USB types (mini B, C, etc.), typical power barrel types, or a proprietary type. This proprietary type could include locating assisters, such as magnets, slots, fins or other types.

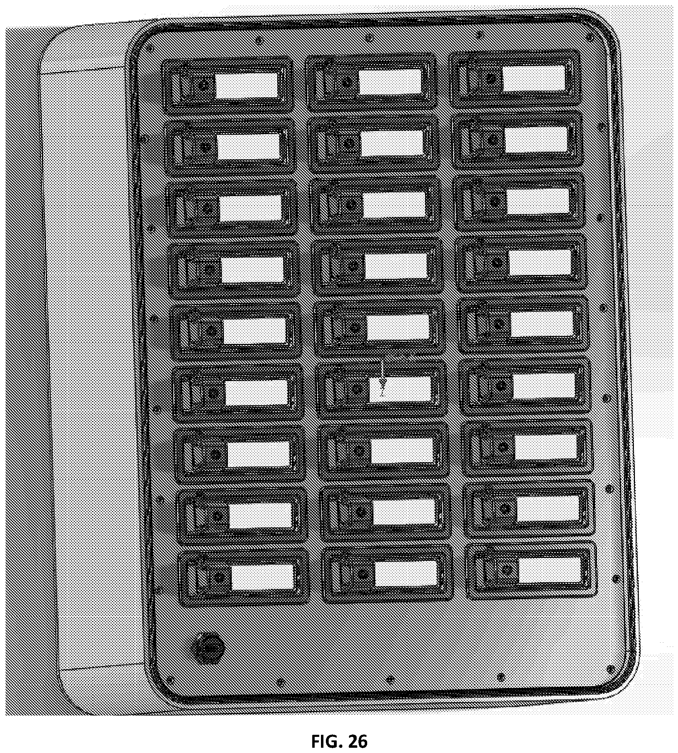

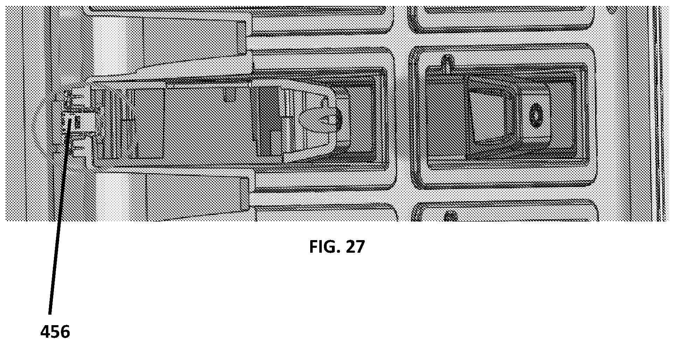

The power connector of the charging case or strap charger may also be designed to mate with a charging sleeve or slot. As shown in FIG. 26, a charging case that may be used to power and charge 27 separate mouthguards. Each slot may include a connector at the back that the ICC may plug into and draw power from. The slot may align the case as it is inserted to ensure the receptacle 454 in the case (e.g. USB micro B or USB C or other) aligns with the plug 456 in the slot, as shown in the cross section in Figure 27. The plug may automatically be inserted all the way when the case is plugged all the way into the slot. In this way, the case may be powered automatically when inserted into the slot.

Different mouthguards (custom or boil and bite) may require different applied wireless charging power, due to size and alignment variations. For this reason, the ICC may have varying, adjustable, or automatically adjustable power outputs to deal with varying alignments and air gaps between Rx/Tx, while helping to ensure that the oral appliance is not subject to excessive heat. An active sensor system on the mouthguard may check this charging temperature and adjust wireless charging at the Rx or Tx accordingly.

The team case may provide peripheral power to such devices that may be useful to its application, such as devices that interface digitally with the mouthguards (iPad, iPhone, other digital devices, etc.) These may include USB or other power connectors, to charge such devices.

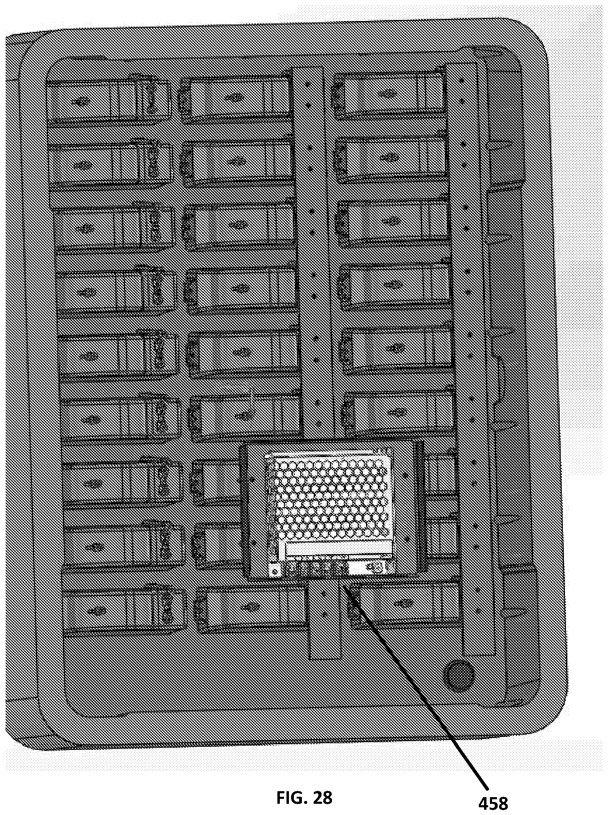

The power connectors (e.g. USB micro B or USB C or other) in the slots or sleeves may be connected to the main power supply with wire harnesses. Alternatively, the plugs in the slots or sleeves may also be connected to the power supply by the use of power distribution bus boards 458, as shown in Figure 28. The bus boards may be any combination of rigid PCB, rigid PCB with flexible sections, rigid-flex-PCB, or rigid boards with wire connectors such as ribbon cable. Assembly may be simpler in this embodiment since multiple wire harnesses need not be made to connect multiple USB plugs directly to the power supply. The charging case power connectors may be mechanically mounted and soldered directly to the bus board, and the bus board may have circuit traces routed to distribute power to all of the individual power connectors. The power supply shown at the center of Figure 28 may be connected to main power and may rectify, regulate and condition this power to provide a lower voltage (e.g. 5VDC for USB) directly to the power connectors via the bus boards. The bus boards may be directly connected to the power supply, or they may be connected via wire harnesses between the power supply and the bus boards (not shown).

The individual charging case may include a UV LED, intended to give the mouthguard and interior of the mouthguard case a sanitizing dose of UV light. This dose may be timed so that it is effective but does not adversely affect the plastics of the mouthguard or the power consumption of the wireless charging process.

The charging case or strap charger may communicate status via an indicator (LED(s), LCD screen, audible buzzer, Bluetooth or other signal, and/or other method(s)) on the user-observable surface of the charger. This indicator may communicate charging status (error, alignment issue, actively charging, overheat, maintenance mode, charging complete, UV cycle. or other status) or communicate other useful information (such as getting a backscatter modulation signal that lets the charger know that the mouthguard's microprocessor is active, even though its Bluetooth communication may have failed, or other information). This communication may also be issued wirelessly to a user interface device, or via wire to the team case or other external user interface.

In some embodiments, the power connectors of the ICCs and/or the slots may also communicate data. In some embodiments, this communication may be via the data pins of a USB connector. In some embodiments, data communicated may include diagnostic data about the mouthguard status, including, for example, charging status. In some embodiments, data communicated may include commands and responses to and from the ICCs from a main controller inside the team case.

In some embodiments, the charger circuit of the ICC and the mouthguard circuit may communicate between themselves such things as charging status (very low battery, overcharge, over-temperature fault, end-of-charge, etc.), status of the microcontroller, or other data. This may be accomplished via Bluetooth, Bluetooth Low Energy, NFC, other digital communication methods, or backscatter modulation, whereby the mouthguard modulates the amount of current it draws (by an internal process that is wasteful on purpose, or by switching a wireless power component in and out of the circuit) at a frequency that is recognized and 'read' by the charger/ power transmitter via the rate(s) of the changing current draw. By this method, a mouthguard may be communicated with, even if its wireless transmitting capability is damaged or compromised.

In some embodiments, the power supply within the team case may include a battery such that the team case may operate and charge mouthguards when not plugged into main power. The team case battery may be charged by plugging the team case into main power and the team case output power to the charging cases may automatically switch over to battery power when main power is disconnected.

In some embodiments, the team case may serve as a communication hub for the mouthguards and/or other wearable electronics devices. The case may include a device for receiving and transmitting information over Bluetooth, Bluetooth Low Energy, NFC, other digital communication methods, or other methods, to communicate with individual wearable devices. The same device, or a different device may transmit this information from the case to the cloud via Wifi, cell, or other longer-range communication methods. This system may serve as a communication hub, without need of human interaction, that can transmit device data, apply firmware updates, and run diagnostics test, among other data transmission tasks where it serves as a general bus between individual local wearable devices and a cloud-based management system.

The team case may be designed to be water tight such that it may be operated outdoors in all weather. It may be designed, for example, as part of a rolling suitcase, such as a Pelican case, that provides a water tight seal between the lid and the base. It may also be designed with a water tight power inlet for main power.

In some embodiments, especially with custom mouthguards, a rare individual dentition may result in a mouthguard that will not fit into an ICC. In this embodiment, a charger circuit may be affixed over the receiver coil of the mouthguard in a modified form. This form may take the shape of a charging circuit (which may be much smaller physically due to it not having to mechanically fit into an ICC). The form may be injection molded into a protective enclosure, with a strap or other retaining feature to keep it aligned with the mouthguard's receiving coil.

Real-time or near-real-time communications from the oral appliance may be desirable for diagnostic purposes. In some cases, data specific to the wearer such as impact dose or other biometric data may be desired to be communicated quickly to personnel such as a coach or athletic trainer. The data may be communicated to a local device for display or it may first be communicated to the internet and then downloaded by a display device. In still other cases, the data may be transmitted to a local device for display or processing and then sent to a mobile device, for example.

There are several standard, de-facto standard, and proprietary wireless communication methods that may be suitable for near-real-time transmission of smart mouthguard data. Bluetooth, Wi-Fi, LORA, LTE, GSM, Zigbee, 802.15.4 and their variants represent some of these methods. There may be tradeoffs in terms of the amount of data that is desired to be sent, the desired transmission time, the overall transmission distance, and the energy required to send the data. Wireless communication methods may be used as point to point communication methods to communicate between the mouthguard, and a tablet computer or other computing device, for example. The system may also provide for communication between mouthguards on the field or between mouthguards and other devices on the field, where messages may be relayed back to one or several data receivers. Communication and relays between such devices may be referred to as a "mesh" communication architecture. Mesh architectures may increase the effective range of communication to farther devices when other devices are nearer the data receiver and can relay the communication back and forth.

Bluetooth in general, and specifically Bluetooth Low Energy (BLE), represents one method to transmit data to and from the mouthguard. Bluetooth Low Energy has very simple provisioning requirements relative to some other wireless connection and provisioning schemes. A BLE connection can be established without a passcode, meaning that smart mouthguards could very simply be connected to display devices such as tablet computers, smartphones, or other computing devices. BLE, as the name indicates, is also capable of operating with lower energy requirements than some other wireless protocols. Lower energy use may be well suited for a smart mouthguard application to reduce the required size of the battery and/or improve the battery life of the mouthguard for each charge.

One feature of BLE that allows low power operation is the advertising mechanism. BLE devices are detected through a procedure based on broadcasting advertising packets. The advertising device regularly sends a packet with a repetition period called the advertising interval. The advertising device (e.g. a smart mouthguard), may put some amount of data into the advertising packet as well. For example, an advertising mouthguard may include a notification that the mouthguard has new data (for example, from a new head impact or biometric test) within the advertising packet. The advertising mouthguard may also include a summary of the conditions of the impact or biometric data, or "summary data", within the advertising packet. The receiving device (e.g. a tablet computer) may receive the advertising packet and obtain all the information of interest from the advertisement. Alternatively, the receiving device may create a connection to the advertising mouthguard to obtain additional information (e.g. raw data from sensors instead of summary data). In a connected mode, the advertising device and receiving device form a dedicated connection, with transmission and reception activated for longer periods of time as long as the connection is maintained. This may increase the power consumption of both devices, and also may block other receiving devices from being able to connect to the advertising mouthguard as long as the connection is maintained. For these reasons, operating in a connected mode may be reserved for those times where the receiving device would like to request more information than can be contained in the advertising packet.

An advertising mouthguard may be discoverable by a receiving device when it is advertising. Therefore, selection of an advertising interval may attempt to strike a balance between latency, or how long it might take the receiving device to detect and receive the advertised data, and power consumption, because advertising requires energy from the advertising device. A high advertising rate may mean that advertised events from the advertising mouthguard make it to the receiving device faster, on average, but may also use more energy from the mouthguard, reducing its battery life. A lower advertising rate may save energy and extend the battery life, but may increase the latency and average delay of the receiving device receiving the information in the advertising packet.

A dynamic advertising interval may be employed as part of attempting to optimize the tradeoff between energy use and latency. In this case, the mouthguard may advertise at a specific less frequent rate in normal operation, but then may increase its advertising rate when it has new information to communicate. For example, the advertising rate may be 5 seconds when there is no new information, but may immediately increase to 1 second intervals when new information is available. In this way, a balance may be struck between maximizing battery life and latency of reporting.

If an impact occurs when there is no receiving device within range to connect to the mouthguard, the mouthguard may try to continue to advertise at the more frequent impact rate, using extra battery life even though the latency is not a significant concern since there are no receiving devices present. For reasons such as this, the advertising mouthguard may choose to revert to its original advertising rate if no receiving device has connected to it within a certain time period after it has increased its advertising rate. This may help to preserve battery life.

Even when there is no impact, the advertising interval may be reduced to save energy if one or more events have not occurred within a certain time period. For example, if there has been no new information, if there have been no connections established by a receiving device, if there have been no new parameter updates, or if there has been no motion within a certain time period, the advertising device may choose to reduce its advertising rate to save energy.

The Bluetooth standard and some other wireless communication standards also allow mesh networks. In these networks, nodes may serve as relays to receive and retransmit communications from other devices. For example, on an athletic field, mouthguards closer to a receiving device, such as a tablet computer, smartphone, or other computing device, may serve as a relay to communicate data from mouthguards that are out of receiving range of the receiving device. In some cases, these mesh networks can be self-forming, where any device may choose to operate as a relay node at any time. In such cases, any player's mouthguard may dynamically choose to relay messages from other mouthguards. The messages may be relayed to the receiving device or through yet another relay node.

In some cases, it may be useful to allow only certain devices to operate as a relay node. For example, referees on the field may have the only relay nodes on the field, since they are unlikely to experience an impact. A relay node on the referee may be worn elsewhere on the body other than in the mouth like a smart mouthguard.

In some cases, a relay node may be worn on the body of a player, since the transmission range through the mouth may be compromised by the player's head. In such cases, the mouthguard may transmit reliably to a relay node on the player's body, and that relay point may transmit the mouthguard's data back to the receiving device.

It may be of interest to know precisely where an athlete was when their mouthguard registered an impact. This may inform the coach or athletic trainer on the nature of the impact and possibly whether it was a true impact during gameplay or a false impact (e.g. mouthguard was dropped on the ground). The tablet PC or other computing device may include GPS location or Wi-fi based location features, and may be able to "geotag" the impact based on when it occurred, where the tablet PC was located when the impact occurred and where the mouthguard was in relation to the tablet when the impact occurred. The mouthguard itself may also have geographical location awareness, whether through GPS, Wireless signals such as Wi-Fi, triangulation of location relative to beacons of known position or proximity to a location-enabled tablet computer.

Smart mouthguards may be associated or assigned to athletes within the system. In this way, the display device may be able to associate data from a smart mouthguard to a specific user. Mouthguards may have unique identification numbers or codes that are stored, displayed or accessed in the form of printed numbers, bar codes, QR codes, RFID tag, radio broadcast, or other methods. In order to associate the mouthguard with a user, an app may be used on a tablet PC or other computing device. The user's information may be entered in the app, or downloaded from a network or local file. The mouthguard identifier code may then be entered or scanned to associate the specific mouthguard with that user.

Although a flowchart or block diagram may illustrate a method as comprising sequential steps or a process as having a particular order of operations, many of the steps or operations in the flowchart(s) or block diagram(s) illustrated herein can be performed in parallel or concurrently, and the flowchart(s) or block diagram(s) should be read in the context of the various embodiments of the present disclosure. In addition, the order of the method steps or process operations illustrated in a flowchart or block diagram may be rearranged for some embodiments. Similarly, a method or process illustrated in a flow chart or block diagram could have additional steps or operations not included therein or fewer steps or operations than those shown. Moreover, a method step may correspond to a method, a function, a procedure, a subroutine, a subprogram, etc.

As used herein, the terms "substantially" or "generally" refer to the complete or nearly complete extent or degree of an action, characteristic, property, state, structure, item, or result. For example, an object that is "substantially" or "generally" enclosed would mean that the object is either completely enclosed or nearly completely enclosed. The exact allowable degree of deviation from absolute completeness may in some cases depend on the specific context. However, generally speaking, the nearness of completion will be so as to have generally the same overall result as if absolute and total completion were obtained. The use of "substantially" or "generally" is equally applicable when used in a negative connotation to refer to the complete or near complete lack of an action, characteristic, property, state, structure, item, or result. For example, an element, combination, embodiment, or composition that is "substantially free of" or "generally free of" an element may still actually contain such element as long as there is generally no significant effect thereof.

In the foregoing description various embodiments of the present disclosure have been presented for the purpose of illustration and description. They are not intended to be exhaustive or to limit the invention to the precise form disclosed.

## Claims

1. An oral appliance (100), comprising:
a generally u-shaped base portion (102) having a tray (110) and a labial flange (112), the labial flange (112) having a plurality of recesses (118) sized and shaped to receive corresponding circuitry components (104) in a force fit;
circuitry (104) arranged on the labial flange (112) and having components arranged in respective recesses (118) of the plurality of recesses (118); and
a cover portion (308) sealingly arranged on the labial flange (112) and covering the circuitry (104).

2. The oral appliance (100) of claim 1, wherein the circuitry (104) is in a registered position on the labial flange (112).

3. The oral appliance of claim 2, wherein the labial flange (112) and the circuitry (104) include alignment features to provide for the registered position.

4. The oral appliance of claim 1, further comprising a dentition portion (309) arranged on the tray (110).

5. The oral appliance of claim 4, wherein the tray (110) comprises a plurality of openings (116) and the dentition portion (309) passes through the plurality of openings (116) to engage bottom teeth of a user.

6. The oral appliance of claim 1, wherein the circuitry (104) comprises a plurality of rigid portions (124) supporting circuitry components and a flexible portion arranged between the plurality of rigid components (124).

7. The oral appliance of claim 1, wherein the circuitry (104) is arranged with substantially all of the components facing the labial flange (112).

8. The oral appliance of claim 1, wherein a portion of the labial flange (112) is a transparent portion.

9. The oral appliance of claim 8, comprising one of the following:
a proximity sensor arranged in registration with the transparent portion; or
a plurality of proximity sensors arranged in registration with the transparent portion, wherein each sensor of the plurality of proximity sensors is arranged at a different position along the u-shaped tray.

10. A method of manufacturing an oral appliance (100), comprising:
establishing a dentition geometry;
forming a first portion (102) of the oral appliance (100) including forming a plurality of recesses (118) sized and shaped to receive corresponding components of circuitry (104) in a force fit;
securing circuitry (104) to the first portion (102) by force fitting the corresponding components of circuitry into respective recesses (118) of the plurality of recesses (118);
forming a second portion (308) and sealing the second portion (308) to the first portion to hermetically seal the circuitry between the first portion (102) and the second portion (308).

11. The method of claim 10, comprising one or more of the following:
wherein the first portion comprises a base portion and securing the circuitry comprises adhering the circuitry to the base portion with substantially all of the components of circuitry facing toward the base portion; and
wherein the second portion comprises a cover portion and securing the circuitry comprises adhering the circuitry to the cover portion with substantially all of the components of circuitry facing toward the cover portion.

12. The method of claim 10, wherein securing the circuitry to the first portion comprises registering the circuitry with the first portion using alignment features.

13. The method of claim 10, wherein forming a first portion comprises thermoforming the first portion.

14. The method of claim 13, wherein thermoforming comprises injection molding.

15. The method of claim 10, wherein forming the first portion comprises additive manufacturing or milling.

16. The method of claim 10, wherein establishing a dentition geometry comprises defining a size and a shape of a tray and a labial flange to accommodate a broad range of users; or scanning a mouth of a user.

## Patentansprüche

1. Ein Mundgerät (100), das Folgendes beinhaltet:
einen allgemein u-förmigen Basisabschnitt (102), der einen Boden (110) und einen labialen Flansch (112) aufweist, wobei der labiale Flansch (112) eine Vielzahl von Aussparungen (118) aufweist, die bemessen und geformt sind, um entsprechende Schaltungskomponenten (104) durch Einpressen aufzunehmen;
eine Schaltung (104), die auf dem labialen Flansch (112) angeordnet ist und Komponenten aufweist, die in jeweiligen Aussparungen (118) der Vielzahl von Aussparungen (118) angeordnet sind; und
einen Abdeckungsabschnitt (308), der abdichtend auf dem labialen Flansch (112) angeordnet ist und die Schaltung (104) abdeckt.

2. Mundgerät (100) gemäß Anspruch 1, wobei die Schaltung (104) in einer registrierten Position auf dem labialen Flansch (112) liegt.

3. Mundgerät gemäß Anspruch 2, wobei der labiale Flansch (112) und die Schaltung (104) Ausrichtungsmerkmale einschließen, um für die registrierte Position zu sorgen.

4. Mundgerät gemäß Anspruch 1, das ferner einen Zahnungsabschnitt (309) beinhaltet, der auf dem Boden (110) angeordnet ist.

5. Mundgerät gemäß Anspruch 4, wobei der Boden (110) eine Vielzahl von Öffnungen (116) aufweist und der Zahnungsabschnitt (309) durch die Vielzahl von Öffnungen (116) geht, um untere Zähne eines Benutzers in Eingriff zu nehmen.

6. Mundgerät gemäß Anspruch 1, wobei die Schaltung (104) eine Vielzahl von starren Abschnitten (124), die die Schaltungskomponenten abstützen, und einen flexiblen Abschnitt, der zwischen der Vielzahl von starren Komponenten (124) angeordnet ist, beinhaltet.

7. Mundgerät gemäß Anspruch 1, wobei die Schaltung (104) mit im Wesentlichen allen Komponenten dem labialen Flansch (112) zugewandt angeordnet ist.

8. Mundgerät gemäß Anspruch 1, wobei ein Abschnitt des labialen Flansches (112) ein transparenter Abschnitt ist.

9. Mundgerät gemäß Anspruch 8, das eines von Folgendem beinhaltet:
einen Näherungssensor, der in Registrierung mit dem transparenten Abschnitt angeordnet ist; oder
eine Vielzahl von Näherungssensoren, die in Registrierung mit dem transparenten Abschnitt angeordnet sind, wobei jeder Sensor der Vielzahl von Näherungssensoren an einer anderen Position entlang des u-förmigen Bodens angeordnet ist.

10. Ein Verfahren zum Fertigen eines Mundgeräts (100), das Folgendes beinhaltet:
Herstellen einer Zahnungsgeometrie;
Bilden eines ersten Abschnitts (102) des Mundgeräts (100), das das Bilden einer Vielzahl von Aussparungen (118) umfasst, die bemessen und geformt sind, um entsprechende Schaltungskomponenten (104) durch Einpressen aufzunehmen;
Sichern der Schaltung (104) am ersten Abschnitt (102) durch Einpressen der entsprechenden Schaltungskomponenten in jeweilige Aussparungen (118) der Vielzahl von Aussparungen (118);
Bilden eines zweiten Abschnitts (308) und Abdichten des zweiten Abschnitts (308) am ersten Abschnitt, um die Schaltung zwischen dem ersten Abschnitt (102) und dem zweiten Abschnitt (308) hermetisch abzudichten.

11. Verfahren gemäß Anspruch 10, das eines oder mehrere von Folgendem beinhaltet:
wobei der erste Abschnitt einen Basisabschnitt beinhaltet und das Sichern der Schaltung das Verkleben der Schaltung mit dem Basisabschnitt mit im Wesentlichen allen Komponenten der Schaltung dem Basisabschnitt zugewandt beinhaltet; und
wobei der zweite Abschnitt einen Abdeckungsabschnitt beinhaltet und das Sichern der Schaltung das Verkleben der Schaltung mit dem Abdeckungsabschnitt mit im Wesentlichen allen Komponenten der Schaltung dem Abdeckungsabschnitt zugewandt beinhaltet.

12. Verfahren gemäß Anspruch 10, wobei das Sichern der Schaltung am ersten Abschnitt das Registrieren der Schaltung mit dem ersten Abschnitt unter Verwendung von Ausrichtungsmerkmalen beinhaltet.

13. Verfahren gemäß Anspruch 10, wobei das Bilden eines ersten Abschnitts das Thermoformen des ersten Abschnitts beinhaltet.

14. Verfahren gemäß Anspruch 13, wobei das Thermoformen das Spritzgießen beinhaltet.

15. Verfahren gemäß Anspruch 10, wobei das Bilden des ersten Abschnitts additives Fertigen oder Fräsen beinhaltet.

16. Verfahren gemäß Anspruch 10, wobei das Herstellen einer Zahnungsgeometrie das Definieren einer Größe und einer Form eines Bodens und eines labialen Flansches, um ein breites Spektrum von Benutzern zu unterstützen; oder das Scannen eines Mundes eines Benutzers beinhaltet.

## Revendications

1. Un appareil buccal (100), comprenant :
une portion formant base (102) généralement en forme de u ayant un plateau (110) et un rebord labial (112), le rebord labial (112) ayant une pluralité de renfoncements (118) dimensionnés et configurés afin de recevoir des composants de circuiterie (104) correspondants dans un montage à force ;
une circuiterie (104) disposée sur le rebord labial (112) et ayant des composants disposés dans des renfoncements (118) respectifs de la pluralité de renfoncements (118) ; et
une portion formant couvercle (308) disposée de façon scellée sur le rebord labial (112) et recouvrant la circuiterie (104).

2. L'appareil buccal (100) de la revendication 1, dans lequel la circuiterie (104) est dans une position repérée sur le rebord labial (112).

3. L'appareil buccal de la revendication 2, dans lequel le rebord labial (112) et la circuiterie (104) incluent des attributs d'alignement permettant d'obtenir la position repérée.

4. L'appareil buccal de la revendication 1, comprenant en outre une portion pour dentition (309) disposée sur le plateau (110).

5. L'appareil buccal de la revendication 4, dans lequel le plateau (110) comprend une pluralité d'ouvertures (116) et la portion pour dentition (309) traverse la pluralité d'ouvertures (116) afin de se mettre en prise avec des dents du bas d'un utilisateur.

6. L'appareil buccal de la revendication 1, dans lequel la circuiterie (104) comprend une pluralité de portions rigides (124) supportant des composants de circuiterie et une portion flexible disposée entre la pluralité de composants rigides (124).

7. L'appareil buccal de la revendication 1, dans lequel la circuiterie (104) est disposée avec substantiellement tous les composants faisant face au rebord labial (112).

8. L'appareil buccal de la revendication 1, dans lequel une portion du rebord labial (112) est une portion transparente.

9. L'appareil buccal de la revendication 8, comprenant l'un des suivants :
un capteur de proximité disposé en repérage avec la portion transparente ; ou
une pluralité de capteurs de proximité disposés en repérage avec la portion transparente, dans lequel chaque capteur de la pluralité de capteurs de proximité est disposé à une position différente le long du plateau en forme de u.

10. Un procédé de fabrication d'un appareil buccal (100), comprenant :
l'établissement d'une géométrie de dentition ;
le formage d'une première portion (102) de l'appareil buccal (100) incluant le formage d'une pluralité de renfoncements (118) dimensionnés et configurés afin de recevoir des composants de circuiterie (104) correspondants dans un montage à force ;
la fixation d'une circuiterie (104) à la première portion (102) par montage à force des composants de circuiterie correspondants à l'intérieur de renfoncements (118) respectifs de la pluralité de renfoncements (118) ;
le formage d'une deuxième portion (308) et le scellement de la deuxième portion (308) à la première portion afin de sceller hermétiquement la circuiterie entre la première portion (102) et la deuxième portion (308).

11. Le procédé de la revendication 10, comprenant un ou plusieurs des énoncés suivants :
dans lequel la première portion comprend une portion formant base et la fixation de la circuiterie comprend le fait de faire adhérer la circuiterie à la portion formant base avec substantiellement tous les composants de circuiterie tournés vers la portion formant base ; et
dans lequel la deuxième portion comprend une portion formant couvercle et la fixation de la circuiterie comprend le collage de la circuiterie à la portion formant couvercle avec substantiellement tous les composants de circuiterie tournés vers la portion formant couvercle.

12. Le procédé de la revendication 10, dans lequel la fixation de la circuiterie à la première portion comprend le repérage de la circuiterie avec la première portion à l'aide d'attributs d'alignement.

13. Le procédé de la revendication 10, dans lequel le formage d'une première portion comprend le thermoformage de la première portion.

14. Le procédé de la revendication 13, dans lequel le thermoformage comprend le moulage par injection.

15. Le procédé de la revendication 10, dans lequel le formage de la première portion comprend une fabrication additive ou un fraisage.

16. Le procédé de la revendication 10, dans lequel l'établissement d'une géométrie de dentition comprend la définition d'une taille et d'une configuration d'un plateau et d'un rebord labial pour une adaptation à un large éventail d'utilisateurs ; ou l'analyse par balayage de la bouche d'un utilisateur.
